# EUROPEAN PATENT SPECIFICATION

(11) **EP 4 015 510 B1**
(45) Date of publication and mention of the grant of the patent: **20.08.2025**
(21) Application number: 19941416.0
(22) Date of filing: 10.10.2019
(51) Int. Cl.: C07D 277/82, A61P 35/00, A61K 31/428

(54) **ANTI-TUMOUR COMPOUND BASED ON JWA GENE ACTIVATION AND HER2 DEGRADATION, PREPARATION METHOD THEREFOR, AND USE THEREOF**
ANTITUMORVERBINDUNG AUF DER GRUNDLAGE VON JWA-GENAKTIVIERUNG UND HER2-ABBAU, VERFAHREN ZU IHRER HERSTELLUNG UND IHRE VERWENDUNG
COMPOSÉ ANTITUMORAL BASÉ SUR L'ACTIVATION DU GÈNE JWA ET LA DÉGRADATION DE HER2, SON PROCÉDÉ DE PRÉPARATION ET SON UTILISATION

(30) Priority: 12.08.2019 CN 201910739357
(43) Date of publication of application: 22.06.2022
(73) Proprietor: Simcere Pharmaceutical Co. Ltd., Jiangbei New District, Nanjing Jiangsu 210032 (CN)
(72) Inventor: ZHOU, Jianwei, Nanjing, Jiangsu 211166 (CN); REN, Yanlin, Nanjing, Jiangsu 211166 (CN); CHEN, Dongyin, Nanjing, Jiangsu 211166 (CN); HUANG, Yefei, Nanjing, Jiangsu 211166 (CN); LI, Aiping, Nanjing, Jiangsu 211166 (CN)
(74) Representative: Potter Clarkson
(86) International application number: PCT/CN2019/110428
(87) International publication number: WO 2021/027045

(56) References cited:
- EP-A1- 0 029 940
- DAYAM R ET AL: "Recent advances in the design and discovery of small-molecule therapeutics targeting HER2/neu", EXPERT OPINION ON THERAPEUTIC PATENTS, TAYLOR & FRANCIS, GB, vol. 17, no. 1, 1 January 2007 (2007-01-01), pages 83 - 102, XP002459515, ISSN: 1354-3776, DOI: 10.1517/13543776.17.1.83
- DATABASE Registry 10 June 2019 (2019-06-10), ANONYMOUS: "N 2327864-42-4; RN 1170955-20-0; RN 1170178-38-7; RN 862974-41-2; RN 862974-32-1; RN 862974-31-0", XP009526065, retrieved from Registry Database accession no. RN 2327864-42-4; RN 1170955-20-0; RN 1170178-38-7; RN 862974-41-2; RN 862974-32-1; RN 862974-31-0
- MANISH CHAUDHARY, DEEPAK PAREEK, PAWAN K. PAREEK, RAVI KANT, KRISHAN G OJHAB, ARUN PAREEK: "Synthesis of some biologically active benzothiazole derivatives", DER PHARMA CHEMICA, vol. 2, no. 5, 31 December 2010 (2010-12-31), pages 281 - 293, XP055780997, ISSN: 0975-413X

## Description

### Technical field

The present invention relates to antitumor compounds, which play an antitumor role based on activation of JWA gene and degradation of HER2. Meanwhile, it also relates to a preparation method and application of the compounds, and belongs to the field of antitumor drugs.

### Background

Malignant tumor (cancer) is a major public health problem that seriously threatens the health of Chinese population. According to the statistical data released by the National Cancer Center in 2019, malignant tumor deaths account for 23.91% of the total causes of death of residents, and the incidence and mortality of malignant tumors have continued to rise in recent 10 years. The annual medical cost caused by malignant tumors exceeds 220 billion, and the situation of prevention and control is grim. Compared with historical data, the cancer burden continues to rise. Over the past 10 years, the incidence of malignant tumors has maintained an annual increase of about 3.9%, and the mortality has maintained an annual increase of 2.5%. Lung cancer, liver cancer, upper digestive system tumors, colorectal cancer, female breast cancer, etc. are still the main malignant tumors in China. Lung cancer ranks first in men, and breast cancer is the leading cause of female morbidity. From age distribution, the incidence of malignant tumors increases with age. The incidence of malignant tumors in young people under 40 years old is at a low level, and increases rapidly from 40 years old onwards. The incidence of malignant tumors is mainly distributed in people over 60 years old, and reaches a peak in age group of 80 years old. The age distribution of different malignant tumors was different. In the past 10 years, the survival rate of malignant tumors has gradually increased. At present, the 5-year relative survival rate of malignant tumors in China is about 40.5%, which is about 10 percentage points higher than that 10 years ago, but there is still a big gap with developed countries. There are gender differences in the incidence of malignant tumors. Lung cancer ranks first in men, with about 520 thousand new cases every year. Other malignant tumors of high incidence are gastric cancer, liver cancer, colorectal cancer and esophageal cancer, respectively. The incidence of the top 10 malignant tumors accounts for about 82.20% of all male malignant tumors. The incidence of breast cancer is the highest in women with incidence rate of about 304 thousand per year. The other major malignant tumors are lung cancer, colorectal cancer, thyroid cancer and gastric cancer, respectively. The incidence of the top 10 female malignant tumors accounts for about 79.10% of all female malignant tumors.

Since the identification of human epidermal growth factor receptor 2 (ERBB2 or HER2) in 1985, the association of human ERBB2 (HER2) oncogene in oncology, especially breast cancer (BC), has been of great concern. The gene encodes 185-kD transmembrane human epidermal growth factor receptor 2 (HER2), a member of the HER1 (or EGFR), HER3, and HER4 receptor families. Activation of their tyrosine kinase domain is usually achieved through homodimerization and heterodimerization induced by a specific ligand. Once activated, cell signaling via family receptors results in cell proliferation and survival. The activation mechanism of HER2 differs from other molecules in that it lacks a specific growth factor ligand and is activated due to its fixed conformation, which is similar to the conditions for ligand activation. This feature makes HER2 be the preferred heterodimeric chaperone for other receptors in the family.

HER2-positive breast cancers (HER2⁺ BC) account for 15-20% of all BCs. Over the past 20 years, the inventions and use directed to monoclonal antibodies (MoAbs), tyrosine kinase inhibitors (TKIs), and antibody-drug conjugates (ADC) to target HER2 have significantly improved prognosis of patients. However, only about 50% of HER2-positive patients respond to targeted therapy, with HER2⁺ metastatic BC (mBC) has little response. Most treatment failures are due to primary or acquired resistance to anti-HER2 therapy. In recent years, multiple mechanisms of drug resistance have been identified, such as damage to drugs that bind to HER2, constitutive activation of signaling pathways parallel to or downstream of HER2, metabolic reprogramming, or reduced immune system activity. Therefore, for HER2-positive patients, it is urgent to find a treatment method that can degrade the overexpressed HER2 protein.

The research team led by Jianwei Zhou, the inventor of this patent application, has been focusing on exploring the structure and function of JWA (also known as ARL6IP5) gene that he discovered and named himself in the past 20 years, especially the relationship between this gene and major human diseases, and has achieved a series of research achievements. JWA gene was found to be a key molecule involved in the regulation of cell differentiation (Chinese Sci Bull, 2001; Chinese Sci Bull, 2002; Biochem Biophys Res Commu, 2006); JWA protein is a cytoskeleton binding protein that has function in regulating cytoskeleton assembly and cell migration (Chinese Sci Bull, 2003; Chinese Sci Bull. 2004; Cell Signal. 2007); JWA is an active environmental response gene and DNA repair protein in normal cells of body tissues (J Biomed Sci, 2005; Free Radic Biol Med, 2007; Nucleic Acids Res, 2009); JWA gene expression level in tissues of multiple cancers such as gastric cancer, melanoma, esophageal cancer and liver cancer was significantly lower than that in adjacent normal tissues; JWA expression level in cancer tissues was positively correlated with patient survival (Oncogene, 2010; Clin Cancer Res, 2012; J Gastroenterol, 2013; Carcinogenesis, 2013; Carcinogenesis, 2014). Studies on cisplatin resistance also found that increasing the expression level of JWA in cisplatin resistant gastric cancer cells can inhibit the phosphorylation level of DNA repair protein XRCC1 and the tolerance of gastric cancer cells to cisplatin by inhibiting the activity of CK2, thus restoring the sensitivity of such cancer cells to cisplatin (Cell Death Dis, 2014). In recent years, JWA has been found to inhibit HER2 expression in gastric cancer cells through ubiquitination modification mechanisms after transcription and translation, which are associated with JWA's inhibition of malignant phenotypes in gastric cancer cells (Oncotarget, 2016a, 2016b). In summary, JWA gene can act as a tumor suppressor gene by acting on multiple targets through a variety of different molecular mechanisms.

At the same time, based on this series of research results, the inventors of this patent application have filed a number of patent applications for invention, including: the patent for invention with the patent application number CN98111422.9 and the issuance number CN1065876C, titled "A specific antibody directed to a protein encoded by cytoskeleton like gene"; the patent for invention with the patent application number CN201310178099.X and the issuance number CN103239710B, titled "A polypeptide having anti-tumor activity of and its use"; the patent for invention with the patent application number CN201610164491.2 and the issuance number CN105820230B, titled "A polypeptide having anti-tumor activity of and its use"; and the patent for invention with the patent application number CN201610857409.4 and the issuance number CN106632299B, titled "An anti-tumor compound, the preparation method and use thereof".

Dayam et al. (Expert Opinion on Therapeutic Patents, vol. 17, no. 1, 1 January 2007) discloses compounds which inhibit the activity of HER2 protein kinase, including those of the quinazoline (e.g., lapatinib), arylazole, benzodithiazole, pyrrolopyridazine, pyrrolotriazine and pyrrolopyrimidine classes of compounds.

### Summary of invention

The main purpose of the invention is to provide anti-tumor compounds for activating JWA gene and degrading HER2 according to the latest research results of the inventors in view of the prior art, which can specifically degrade HER2 through mechanisms such as cascade activation of E3 ubiquitin enzyme upon activation of expression of JWA gene, so as to realize the inhibitory or therapeutic effect on malignant tumors overexpressing HER2. In addition, a preparation method and application of the compounds are provided.

The main technical idea of the invention is as follows: according to the previous series of research results by the inventors, JWA has outstanding biological function of tumor suppressor gene, and it has been verified in experimental intervention models for the occurrence and development of various human malignant tumors. The expression level of JWA protein in most malignant tumor tissues is significantly lower than that in adjacent normal tissues, and JWA protein has effect of inhibiting the overexpression of HER2. Therefore, a reporter gene cell model containing the regulatory region of the JWA gene promoter was designed and constructed by the inventors, and high-throughput screening was conducted to find small molecule compounds that can activate JWA gene expression. If the desired small molecule compound is obtained, its potential anti-tumor biological effect will be validated in cancer cells with high HER2 expression.

With this as the main technical idea, the inventors' research group carried out high-throughput screening in cooperation with the National Compound Library and other institutions. Based on some lead compounds obtained by screening, further targeted molecular structure modification was carried out to synthesize a series of dibenzothiazolamine compounds. It was verified by multiple cell models and animal models that the series of compounds could effectively activate JWA gene expression in cells and tissues and organs of mice *in vivo.*

The present invention is as defined in the claims.

As provided herein, compounds for use in activating JWA gene and degrading HER2 are represented by Formula I: 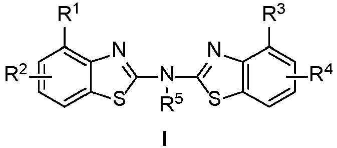 wherein
R¹ is selected from the group consisting of -OH, -CH₃, -CH₂CH₃, -OCH₃, and - OCH₂CH₃;
R² is selected from the group consisting of -H, -OH, -CH₃, -CH₂CH₃, - CH₂CH₂CH₃, -OCH₃, and -OCH₂CH₂CH₃;
R³ is selected from the group consisting of -F, -Cl, -Br, -I, and -CF₃;
R⁴ is selected from the group consisting of -H, -F, -Cl, -Br, -I, and -CF₃; and
R⁵ is selected from the group consisting of -H, -CH₃, and -CH₂CH₃.

The compounds are bis-benzothiazolamine compounds.

Preferably, R¹ is -OCH₃; R² is -H or -OCH₃; R³ is -F, or -CF₃; R⁴ is -H or -F; and R⁵ is -H.

Preferably, the compound of Formula I is selected from the group consisting of: 4-fluoro-N-(4-methoxybenzo[d]thiazol-2-yl)benzo[d]thiazol-2-amine; 4,7-difluoro-N-(4-methoxybenzo[d]thiazol-2-yl)benzo[d]thiazol-2-amine; 4,6-difluoro-N-(4-methoxylbenzo[d]thiazol-2-yl)benzo[d]thiazol-2-amine; 4-methoxy-N-(4-(trifluoromethyl)benzo[d]thiazol-2-yl)benzo[d]thiazol-2-amine; N-(4-fluorobenzo[d]thiazol-2-yl)-4,7-dimethoxybenzo[d]thiazol-2-amine; N-(4-fluorobenzo[d]thiazol-2-yl)-4,6-dimethoxybenzo[d]thiazol-2-amine; 4,7-dimethoxy-N-(4-(trifluoromethyl)benzo[d]thiazol-2-yl)benzo[d]thiazol-2-amine; and 4,6-dimethoxy-N-(4-(trifluoromethyl)benzo[d]thiazol-2-yl)benzo[d]thiazol-2-amine, with the following structural formulae, respectively: 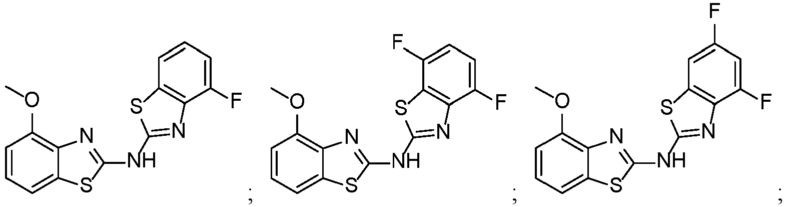 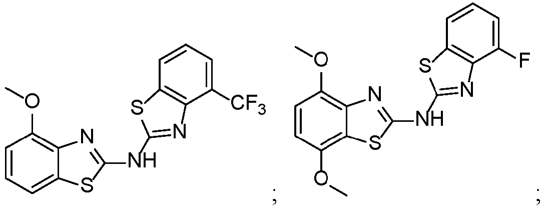 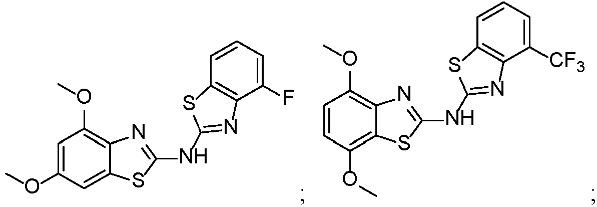 and

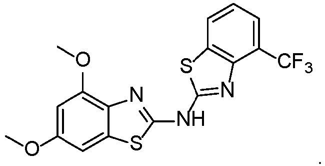

It has been demonstrated by experiments that the above-mentioned compounds are effective in activating the expression of the JWA gene in cells and the tissues and organs of mice. Of particular significance is that the research of the inventors' research group reveals that the above-mentioned antitumor compounds specifically degrade HER2 protein overexpressed in tumor cells through action mechanisms, such as cascade activation of E3 ubiquitin enzyme, and have the functions of inhibiting the proliferation and growth of tumor cells. Meanwhile, the significant antitumor effect of representative compounds of the above-mentioned antitumor compounds was also validated in a tumor-bearing nude mouse model subcutaneously transplanted with human breast cancer cells overexpressing HER2. Particularly, the representative compounds also exhibited a protective effect on the functions of major organs including the heart, liver, and kidney in mice while exerting a significant antitumor effect (see specific examples below for details).

Also provided is:
a method for preparing the above-mentioned compounds, which comprises the following steps: 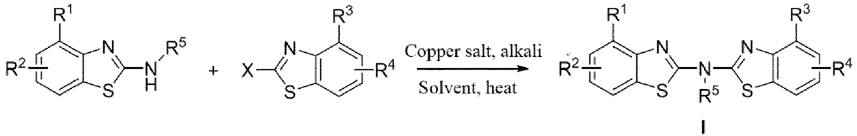
wherein X is selected from the group consisting of Cl, Br, and I; said copper salt is selected from the group consisting of copper oxide, cuprous oxide, copper chloride, cuprous chloride, copper bromide, cuprous bromide and cuprous iodide; said alkali is selected from the group consisting of sodium hydroxide, potassium hydroxide, potassium carbonate, sodium carbonate, potassium tert-butoxide and sodium hydride; said solvent is selected from the group consisting of tetrahydrofuran, dimethylsulfoxide and N,N-dimethylformamide; and said heating temperature is 80-120°C (R¹, R², R³, R⁴, and R⁵ are as defined in the above).

Preferably, X is Cl; said copper salt is cuprous iodide; said alkali is potassium carbonate; said solvent is dimethylsulfoxide; and said heating temperature is 100°C.

Also provided is a pharmaceutical salt of the above-mentioned compounds.

Preferably, the pharmaceutical salt is a salt formed by the compound of formula I with an acid selected from the group consisting of hydrochloric acid, sulfuric acid, benzenesulfonic acid, *p*-toluenesulfonic acid, phosphoric acid, hydrobromic acid, maleic acid, fumaric acid and malic acid.

Also provided is a pharmaceutical composition comprising the above-mentioned compound or the above-mentioned pharmaceutical salt.

Further provided is the above-mentioned compound, or the above-mentioned pharmaceutical salt, or the above-mentioned pharmaceutical composition for use as a JWA gene activator or an antitumor medicament.

Preferably, the tumors are those associated with low expression of JWA gene and overexpression of HER2, and the tumors include breast cancer, gastric cancer, lung cancer, glioma, etc.. The antitumor drug inhibits the growth of the tumors by activating the expression of the JWA protein and then degrading the HER2 protein.

In addition, specific dosage forms of the above-mentioned compounds, pharmaceutical salts and pharmaceutical compositions can be selected according to the prior art in combination with actual requirements, such as tablets, powders, pills, capsules, suppositories, granules, suspensions, oral liquids, injections, and so on. Tablets and capsules for oral administration comprise conventional excipients, such as fillers, lubricants, dispersants, diluents, and binders.

Compared with the prior art, these antitumor compounds and pharmaceutical salts are effective in activating the expression of JWA protein, and further specifically degrading overexpressed HER2 protein through the cascade activation of E3 ubiquitin enzymes and so on, and thereby inhibiting the proliferation and growth in vivo of tumor cells.

An outstanding advantage of these compounds and pharmaceutical salts is to degrade the overexpressed HER2 protein in cancer cells, which are completely different from various anti-HER2 antibodies or inhibitors that inhibit the activity of HER2 protein kinase commonly used in the word to date. With reference to the theory of molecular cell biology, it is clear that the use of the antitumor compounds and pharmaceutical salts thereof of the present invention can be significantly superior to current anticancer therapies for HER2-overexpressing cancers, because the antitumor compounds of the present invention have the effect of ultimately degrading HER2 protein, which can fundamentally eliminate the material basis for the drug-resistance phenomenon caused by the adaptive allostery of HER protein following the use of anti--HER2 antibodies or inhibitors.

The compounds and pharmaceutical salts thereof also have a remarkable therapeutic activity against tumors at a relatively low dosage, and not only are nontoxic to normal tissue cells, but also have a certain protective effect on important organs including the heart, liver, kidney, etc., which have a broad application prospect.

### Brief description of Drawings

FIG. **1** shows the cDNA sequence, amino acids that encode proteins, and the promoter sequence used to construct a reporter gene of JWA gene in accordance with Example **11** of the present invention.
FIG. **2** shows a structure of a reporter gene containing the promoter sequence of JWA gene in accordance with Example **11** of the present invention.
FIG. **3** shows the effect of JAC1 on colony formation of two human breast cancer cells in accordance with Example **12** of the present invention.
FIG. **4** shows the effect of JAC1 on the cellular localization and the expression level of HER2 in two human breast cancer cells in accordance with Example **13** of the present invention.
FIG. **5** shows the effect of JAC1 on activating JWA expression and inhibiting HER2 expression in two human breast cancer cells in accordance with Example **14** of the present invention.
FIG. **6** shows the inhibitory effect of JAC1 on the migration of two human breast cancer cells BT474 and SKBR3 in accordance with Example **15** of the present invention.
FIG. **7** shows a curve graph of body weight gain of mice subcutaneously transplanted with human breast cancer cell BT474 treated with JAC1 in accordance with Example **16** of the present invention.
FIG. **8** shows a curve graph of tumor volume of mice subcutaneously transplanted with human breast cancer cell BT474 treated with JAC1 in accordance with Example **16** of the present invention.
FIG. **9** shows a result of tumor weights of mice subcutaneously transplanted with human breast cancer cell BT474 treated with JAC1 in accordance with Example **16** of the present invention.
FIG. **10** shows a result of tumor weight/body weight ratio of mice subcutaneously transplanted with human breast cancer cell BT474 treated with JAC1 in accordance with Example **16** of the present invention.
FIG. **11** shows a result of the tumor tissues isolated from each group of mice subcutaneously transplanted with human breast cancer cell BT474 in accordance with Example **16** of the present invention.
FIG. **12** shows a result of the tumor growth inhibition rate of each group of mice subcutaneously transplanted with human breast cancer cell BT474 in accordance with Example **16** of the present invention.
FIG. **13** shows the expression levels of related molecules in tumor tissues of each group of mice subcutaneously transplanted with human breast cancer cell BT474 in accordance with Example **16** of the present invention.
FIG. **14** shows a H&E-staining image of major organs of mice subcutaneously transplanted with human breast cancer cells treated with JAC1 in accordance with Example **17** of the present invention.
FIG. **15** shows a blood biochemical result of mice subcutaneously transplanted with human breast cancer cells treated with JAC1 in accordance with Example **18** of the present invention.
FIG. **16** shows a result of off-target effects occurring when JAC1 activates JWA protein and represses HER2 expression in accordance with Example **19** of the present invention.

### Detailed description

The present invention is further described in detail with reference to the attached figures and examples. However, the present invention is not limited to the following examples.

### Example 1

### Preparation of 4-fluoro-N-(4-methoxybenzo[d]thiazol-2-yl) benzo[d]thiazol-2-amine:

To a 50 mL dry eggplant-shaped flask equipped with a spherical reflux condenser and a magnet were added 2-chloro-4-fluorobenzo[d]thiazole (1 g, 5.3 mmol), 4-methoxybenzo[d]thiazol-2-amine (0.96 g, 5.3 mmol), cuprous iodide (0.31 g, 0.3 mmol), potassium carbonate (1.47 g, 10.6 mmol), and dimethylsulfoxide (20 mL), and heated to 100°C and then reacted for 6 hours. After completion of the reaction, the reaction mixture was cooled to room temperature, diluted with water (60 mL), and extracted 3 times with ethyl acetate (30 mL). The combined organic phase was washed with water and saturated brine successively, dried over anhydrous sodium sulfate, and filtered. The solvent was then removed by evaporation under reduced pressure to afford a black solid. The crude product was mixed with silica gel powders, and purified by silica gel column chromatography (eluent: V_{PE}: V_{EA} = 5: 1), to afford a black solid, which was then slurried several times with ethyl acetate or methanol, to obtain a white solid compound (0.7 g, yield: 39%).

¹H NMR (400 MHz, DMSO-d₆) δ 7.75 (d, J = 5.8 Hz, 1H), 7.49 (d, J = 7.9 Hz, 1H), 7.28-7.17 (m, 3H), 7.00 (d, J = 8.0 Hz, 1H), 3.92 (s, 3H).

ESI-MS m/z: 332.1 {[M + H]⁺}.

### Example 2

### Preparation of 4,7-difluoro-N-(4-methoxybenzo[d]thiazol-2-yl) benzo[d]thiazol-2-amine:

The compound in Example **2** was prepared with reference to the method shown in Example **1** by replacing 2-chloro-4-fluorobenzo[d]thiazole with 2-chloro-4,7-difluorobenzo[d]thiazole, to afford an off-white solid (1.1 g, yield: 57%).

¹H NMR (400 MHz, DMSO-d₆) δ 7.73 (d, J = 5.4 Hz, 1H), 7.28-7.17 (m, 3H), 7.02 (d, J = 8.0 Hz, 1H), 3.92 (s, 3H).

ESI-MS m/z: 350.1 {[M + H]⁺}.

### Example 3

### Preparation of 4,6-difluoro-N-(4-methoxylbenzo[d]thiazol-2-yl) benzo[d]thiazol-2-amine:

The compound in Example **3** was prepared with reference to the method shown in Example **1** by replacing 2-chloro-4-fluorobenzo[d]thiazole with 2-chloro-4,6-difluorobenzo[d]thiazole, to afford an off-white solid (0.8 g, yield: 45%).

¹H NMR (400 MHz, DMSO-d₆) δ 7.65 (d, J = 5.8 Hz, 1H), 7.35 (d, J = 7.9 Hz, 1H), 7.28-7.17 (m, 2H), 7.03 (d, J = 8.0 Hz, 1H), 3.92 (s, 3H).

ESI-MS m/z: 350.1 {[M + H]⁺}.

### Example 4

### Preparation of 4-methoxy-N-(4-(trifluoromethyl)benzo[d]thiazol-2-yl) benzo[d]thiazol-2-amine:

The compound in Example **4** was prepared with reference to the method shown in Example **1** by replacing 2-chloro-4-fluorobenzo[d]thiazole with 2-chloro-4-(trifluoromethyl)benzo[d]thiazole, to afford a white solid (1.1 g, yield: 67%).

¹H NMR (400 MHz, DMSO-d₆) δ 7.55 (d, J = 5.8 Hz, 1H), 7.39 (d, J = 7.9 Hz, 1H), 7.15-7.07 (m, 3H), 7.00 (d, J = 8.0 Hz, 1H), 3.92 (s, 3H).

ESI-MS m/z: 382.1 {[M + H]⁺}.

### Example 5

### Preparation of N-(4-fluorobenzo[d]thiazol-2-yl)-4,7-dimethoxy benzo[d]thiazol-2-amine:

The compound in Example **5** was prepared with reference to the method shown in Example **1** by replacing 4-methoxybenzo[d]thiazol-2-amine with 4,7-dimethoxybenzo[d]thiazol-2-amine, to afford a white solid (1.4 g, yield: 73%).

¹H NMR (400 MHz, DMSO-d₆) δ 7.74 (d, J = 5.8 Hz, 1H), 7.39 (d, J = 7.9 Hz, 1H), 7.27-7.15 (m, 3H), 7.00 (d, J = 8.0 Hz, 2H), 3.82 (s, 3H), 3.76 (s, 3H).

ESI-MS m/z: 362.1 {[M + H]⁺}.

### Example 6

### Preparation of N-(4-fluorobenzo[d]thiazol-2-yl)-4,6-dimethoxy benzo[d]thiazol-2-amine:

The compound in Example **6** was prepared with reference to the method shown in Example **1** by replacing 4-methoxybenzo[d]thiazol-2-amine with 4,6-dimethoxybenzo[d]thiazol-2-amine, to afford a white solid (1.2 g, yield: 64%).

¹H NMR (400 MHz, DMSO-d₆) δ 7.75 (d, J = 5.8 Hz, 1H), 7.28-7.17 (m, 2H), 7.21 (s, 1H), 6.79 (s, 1H), 3.88 (s, 3H), 3.83 (s, 3H).

ESI-MS m/z: 332.1 {[M + H]⁺}.

### Example 7

### Preparation of 4,7-dimethoxy-N-(4-(trifluoromethyl)benzo[d]thiazol-2-yl) benzo[d]thiazol-2-amine:

The compound in Example **7** was prepared with reference to the method shown in Example **1** by replacing 2-chloro-4-fluorobenzo[d]thiazole with 2-chloro-4-(trifluoromethyl)benzo[d]thiazole and replacing 4-methoxybenzo[d]thiazol-2-amine with 4,7-dimethoxybenzo[d]thiazol-2-amine, to afford a white solid (0.9 g, yield: 53%).

¹H NMR (400 MHz, DMSO-d₆) δ 7.75 (d, J = 5.8 Hz, 1H), 7.49 (d, J = 7.9 Hz, 1H), 7.28-7.17 (m, 1H), 7.00 (d, J = 8.0 Hz, 2H), 3.92 (s, 3H).

ESI-MS m/z: 412.1 {[M + H]⁺}.

### Example 8

### Preparation of 4,6-dimethoxy-N-(4-(trifluoromethyl)benzo[d]thiazol-2-yl) benzo[d]thiazol-2-amine:

The compound in Example **8** was prepared with reference to the method shown in Example **1** by replacing 2-chloro-4-fluorobenzo[d]thiazole with 2-chloro-4-(trifluoromethyl)benzo[d]thiazole and replacing 4-methoxybenzo[d]thiazol-2-amine with 4,6-dimethoxybenzo[d]thiazol-2-amine, to afford a white solid (0.8 g, yield: 47%).

¹H NMR (400 MHz, DMSO-d₆) δ 7.75 (d, J = 5.8 Hz, 1H), 7.49 (d, J = 7.9 Hz, 1H), 7.28-7.17 (m, 1H), 7.21 (s, 1H), 7.69 (s, 1H), 3.92 (s, 3H)_{∘}

ESI-MS m/z: 412.1 {[M + H]⁺}.

### Example 9

### Preparation of pharmaceutical composition: tablets

1 G of the compound of Formula I or pharmaceutical salt thereof was mixed with 23 g of lactose and 5.7 g of microcrystalline cellulose by using a mixer. The resulting mixture was compressed by using a roller compactor to obtain a sheet-like compressed solid. The sheet-like compressed solid was ground into powders by a hammer mill, and the resulting powders were sieved through a 20-mesh sieve. 0.3 G of light silica and 0.3 g of magnesium stearate were added to the sieved powders, mixed, and pressed into tablets by using a tabletting machine.

As an example, the compound of Formula I was selected from the compounds prepared in Examples **1-8.**

As an example, the acid moiety of the pharmaceutical salt can be derived from hydrochloric acid, sulfuric acid, benzenesulfonic acid, *p*-toluenesulfonic acid, phosphoric acid, hydrobromic acid, maleic acid, fumaric acid, or malic acid.

### Example 10

### Preparation of pharmaceutical composition: gelatin capsules

1 G of the compound of Formula I or pharmaceutical salt thereof was mixed with 0.35g of microcrystalline cellulose and 0.15 g of lactose and then granulated with water. Then, the resulting granules were mixed with 0.04 g of croscarmellose sodium and 0.01 g of magnesium stearate. The resulting mixture were filled into gelatin capsule shells to obtain gelatin capsules (The gelatin capsule shells used in this Example were manufactured by Suzhou Capsule Co., Ltd. in China, and met the pharmaceutical standards).

As an example, the compound of Formula I was selected from the compounds prepared in Examples **1-8.**

As an example, the acid moiety of the pharmaceutical salt can be derived from hydrochloric acid, sulfuric acid, benzenesulfonic acid, *p*-toluenesulfonic acid, phosphoric acid, hydrobromic acid, maleic acid, fumaric acid and malic acid.

### Example 11

### Comparison of biological activities of bis-benzothiazolamine compounds:

The compounds synthesized in Examples **1-8** were named as JAC1, JAC1011, JAC1012, JAC1013, JAC1014, JAC1015, JAC1016, and JAC1017 in sequence.

High-throughput screening was performed on the human bronchial epithelial (HBE) cells stably transfected with a 2,000 bp promoter sequence of JWA gene and a reporter gene having luciferase indicating function. After two rounds of high-throughput screening, the fluorescence intensity of the reporter gene, the expression of which was influenced by the eight representative compounds, was shown in Table 1 at the compound concentration ranging from 0.00 g/mL-3.30 µg/mL and the incubation time of the cells for 48 h. Table **2** showed the Chinese-English comparison of the eight representative compounds.

Referring to Table **1,** the eight compounds activated the expression of JWA gene in a cell and showed a clear dose-effect relationship where JAC1 has a particularly significant effect on activation of JWA gene.

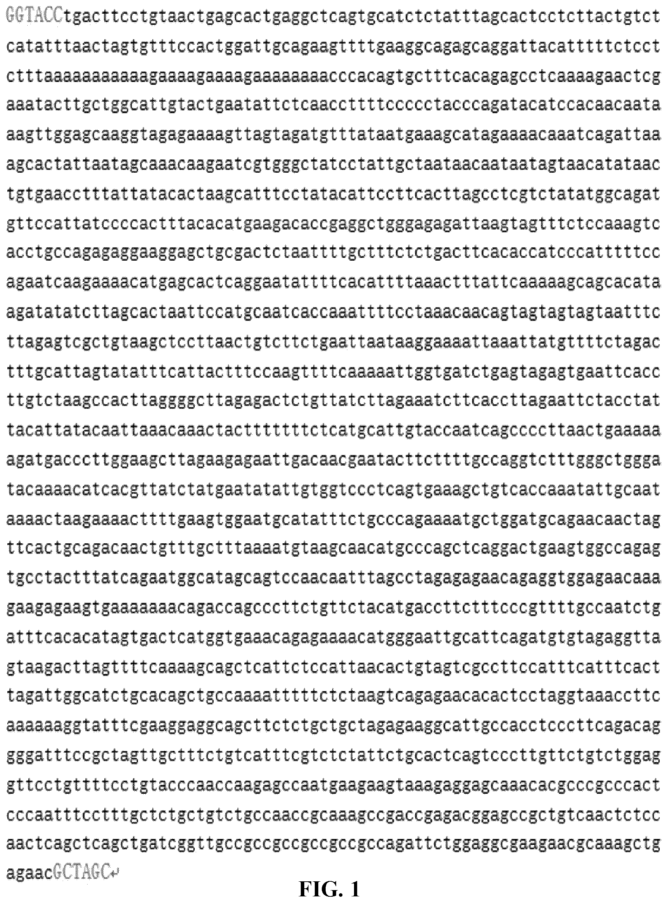

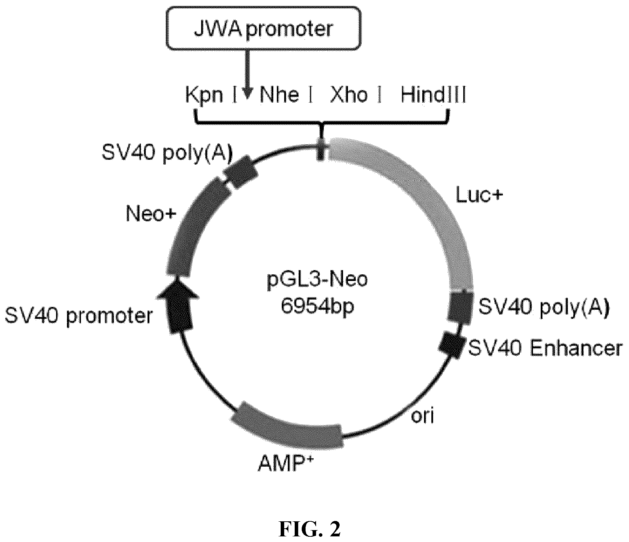

FIG. 1 shows the full-length cDNA sequence and amino acid sequence of encoding protein of JWA gene, and 2000 bp promoter DNA sequence of JWA gene used to construct luciferase reporter gene. FIG. **2** shows the molecular structure of the luciferase reporter gene plasmid containing the promoter sequence of JWA gene.

**Table 1 Bisbenzothiazolamide compounds and fluorescence intensity of reporter gene**

| Concentration µg/mL | JAC1 | JAC1011 | JAC1012 | JAC1013 | JAC1014 | JAC1015 | JAC1016 | JAC1017 |
|---|---|---|---|---|---|---|---|---|
| 0.00 | 12053.33 | 12126.67 | 12580.00 | 12687.67 | 12614.67 | 13050.00 | 12696.67 | 12440.00 |
| 0.05 | 13560.00 | 13255.33 | 13448.67 | 13315.33 | 13835.33 | 14335.33 | 14360.00 | 12910.00 |
| 0.10 | 16200.00 | 13553.33 | 13518.67 | 12346.00 | 14254.00 | 15573.33 | 16238.00 | 13086.67 |
| 0.21 | 21975.00 | 15397.33 | 16205.00 | 14926.00 | 15262.67 | 18260.67 | 19226.00 | 14460.00 |
| 0.41 | 28186.67 | 19835.00 | 19768.67 | 16036.67 | 18680.00 | 22168.67 | 24518.00 | 17188.00 |
| 0.83 | 38760.00 | 25886.67 | 25237.33 | 20014.00 | 22088.00 | 28162.67 | 28746.00 | 22680.00 |
| 1.65 | 54293.33 | 31413.33 | 33228.00 | 26261.67 | 28702.00 | 30168.67 | 32253.33 | 28406.00 |
| 3.30 | 68080.00 | 39676.33 | 41139.33 | 35332.33 | 37664.67 | 37663.33 | 36361.67 | 37426.00 |

**Table 2 Numberings of Bisbenzothiazolamide compounds and Chinese-English names thereof**

| No. | Compounds |
|---|---|
| JAC1 | 4-fluoro-N-(4-methoxybenzo[d]thiazol-2-yl)benzo[d]thiazol-2-amine |
| JAC1011 | 4,7-difluoro-N-(4-methoxybenzo[d]thiazol-2-yl)benzo[d]thiazol-2-amine |
| JAC1012 | 4,6-difluoro-N-(4-methoxylbenzo[d]thiazol-2-yl)benzo[d]thiazol-2-amine |
| JAC1013 | 4-methoxy-N-(4-(trifluoromethyl)benzo[d]thiazol-2-yl)benzo[d]thiazol-2-amine |
| JAC1014 | N-(4-fluorobenzo[d]thiazol-2-yl)-4,7-dimethoxybenzo[d]thiazol-2-amine |
| JAC1015 | N-(4-fluorobenzo[d]thiazol-2-yl)-4,6-dimethoxybenzo[d]thiazol-2-amine |
| JAC1016 | 4,7-dimethoxy-N-(4-(trifluoromethyl)benzo[d]thiazol-2-yl)benzo[d]thiazol-2-amine |
| JAC1017 | 4,6-dimethoxy-N-(4-(trifluoromethyl)benzo[d]thiazol-2-yl)benzo[d]thiazol-2-amine |

### Example 12

Effect of JAC1 on the colony formation (malignant proliferation) of HER2-positive breast cancer cells.

This example is used to determine the effect of treatment with the small-molecule compound JAC1 on the colony formation (malignant proliferation) of human breast cancer cells BT474 and SKBR3 overexpressing HER2 protein.

Two human breast cancer cell lines BT474 and SKBR3 were cultured in DMEM medium containing, respectively, 20% and 10% fetal bovine serum, conventional double antibody, and were placed in a incubator at 37°C, 5% CO₂. The BT474 and SKBR3 cells in the logarithmic growth phase were respectively transferred into 6-well plates at a density of 800 cells per well. Upon the cells were adhered, the cells were cultured in the medium containing different concentrations (0, 1, and 10 µM) of JAC1, respectively. The cells were cultured for 10-15 d and stopped according to growth of cell colonies, followed by removal of medium. After the cell colonies were fixed with methanol, stained with crystal violet, and photographed, the number of the cell colonies (each containing more than 50 cells) was counted using the software ImageJ. The statistical soft SPSS was used for statistical analysis.

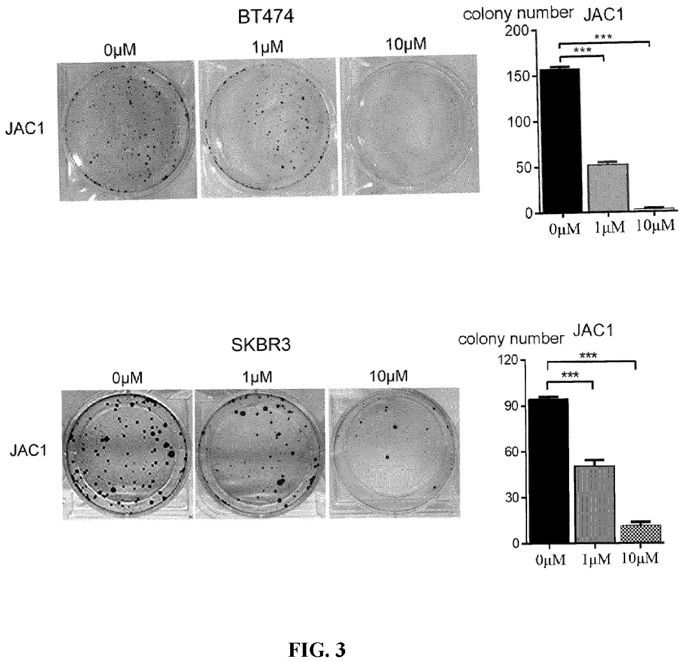

The experimental results are shown in FIG. **3****.** When compared to the control group, different doses of the small-molecule compound JAC1 significantly inhibited the colony-forming ability of BT474 and SKBR3 cells and showed a dose-effect relationship.

It should be noted that: the other compounds prepared in Examples **1-8** were detected with reference to the method of Example **12.** The results were consistent with JAC1 that significantly inhibited the colony-forming ability of BT474 and SKBR3 cells and showed a dose-effect relationship.

### Example 13

Effect of JAC1 on the cellular location of HER2 molecule in HER2-positive breast cancer cells.

This example is used to determine whether the treatment with the small-molecule compound JAC1 affects the cellular localization of HER2 protein in cells.

The cells were seeded into a 35 mm dish for laser confocal scanning microscope and treated with different concentrations (0, 1, and 10 µM) of JAC1 for 24 h. Then, the cells were fixed with methanol for 15 min, washed three times with PBST, blocked with normal sheep serum for 1 h, and incubated with the primary antibody overnight at 4°C. The next day, the cells were washed three times with PBST, incubated with fluorescent secondary antibody and 4',6-diamidino-2-phenylindole (DAPI), and photographed using a laser confocal scanning microscope.

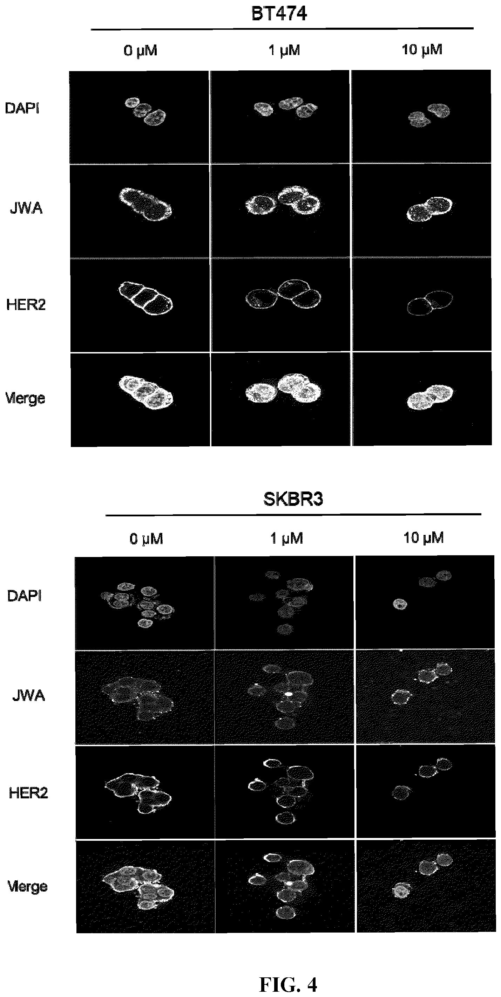

The experimental results are shown in FIG. **4****,** the JWA level increased and the HER2 expression level reduced with increasing concentration of JAC1, and attenuated HER2 was still localized on the cell membrane. In conclusion, the treatment with JAC1 had no effect on the localization of HER2 on the cell membrane.

It should be noted that: the other compounds prepared in Examples **1-8** were detected with reference to the method of Example **13.** The results were consistent with JAC1 that JWA level increased and the HER2 expression level reduced with increasing concentration of the compounds, and attenuated HER2 was still localized on the cell membrane. In conclusion, the treatment with these compounds had no effect on the localization of HER2 on the cell membrane.

### Example 14

Effect of JAC1 on the expression levels of JWA and HER2 proteins in HER2-positive breast cancer cells.

This example is used to verify the effect of treatment with JAC1 on the increase in JWA protein level and the decrease in HER2 protein level by using a cell model.

The cells in the logarithmic growth phase were routinely digested, and seeded into 6-well plates at the cell density of 40%-50%. The cells were treated with different concentrations of JAC1 for 24 h, followed by addition of the cell lysis buffer of 0.1 mL of Radioimmunoprecipitation Assay Butter (RIPA, containing 0.5% phenylmethylsulfonyl fluoride (PMSF)) for protein extraction. After centrifugation at 12,000×g for 15 min, the supernatant was collected. The proteins were tested for the concentration and subjected to boiling. Proteins were subjected to electrophoresis with 10% polyacrylamide gel at 40 µg protein per gel well under the conditions of 60 V for 30 min and 90 V for 1.5-2 h. After gel electrophoresis, the proteins were transferred by the wet method from the gel to a PVDF membrane, and blocked with 5% skim milk at room temperature for 1-2 h. The membrane was washed with Tris-Buffered Saline and Tween 20 (TBST, containing 0.1% Tween 20) three times for 5 min each, and incubated with the corresponding primary antibody overnight at 4°C. The next day, the membrane was washed with the TBST (containing 0.1% Tween 20) three times for 5 min each, and incubated with the secondary antibody at room temperature for 1-2 h. The membrane was washed with the TBST (containing 0.1% Tween 20) eight times for 5 min each. Electrochemiluminescence (ECL) solution was dropwise added to the membrane, followed by exposure.

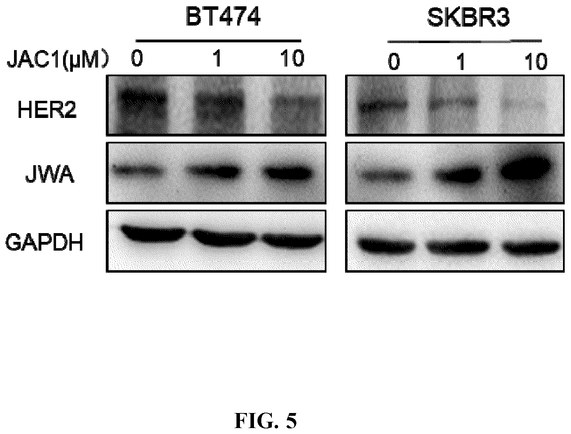

The experimental results are shown in FIG. **5****,** the JWA protein level increased and the HER2 protein level decreased with increasing concentration of JAC1.

It should be noted that: other compounds prepared in Examples **1-8** were detected with reference to the method of Example **14.** The results were consistent with JAC1 that the JWA protein level increased and the HER2 protein level decreased with increasing concentration of each prepared compound.

### Example 15

Effect of JAC1 on the migratory ability of HER2-positive breast cancer cells.

This example is used to determine the effect of JAC1 on the migratory ability of breast cancer cells BT474 and SKBR3 through Transwell migration assay.

BT474 and SKBR3 cells in the logarithmic growth phase were seeded into six-well plates at the cell density of 40%-50%. After adhered, the cells were respectively incubated with 0, 1, and 10 µM of JAC1. Spreading gel in Transwell chamber: on the day before the assay, a 24-well plate and a plurality of Transwell chambers were prepared, and 1 mg/mL fibronectin (FN) mother liquor was diluted 10 times to a final concentration of 100 µg/mL. The bottom of each Transwell chamber was coated with 50 µL of FN, placed on a clean bench to air-dry for 2 h, and cultured in an incubator overnight.

On the day of the assay, the cultured cells were digested with 0.25% trypsin and resuspended in a serum-free medium in which the number of the cells were counted and the cell density was adjusted to 3×10⁵ cells/mL. 100 µL of the resultant culture was transferred onto the upper layer of each Transwell chamber, and 600 µL of medium containing 10% FBS and 100 ng/mL EGF was added to the lower layer of each Transwell chamber. After incubation in an incubator for 12 h, the Transwell chambers were taken out, fixed with 95% methanol for 20 min, washed three times with phosphate-buffered saline (PBS), stained with crystal violet dye for 30 min, and washed three times with PBS. The cells adhered to the Transwell chambers were gently wiped from the upper layer of each Transwell chamber with a cotton swab. Each Transwell chamber was placed on a glass slide, observed using an inverted microscope through which images were photographed on the top, bottom, left, right and middle of each Transwell chamber. The number of the cells passing through the Transwell chamber was counted and the statistical analysis was performed.

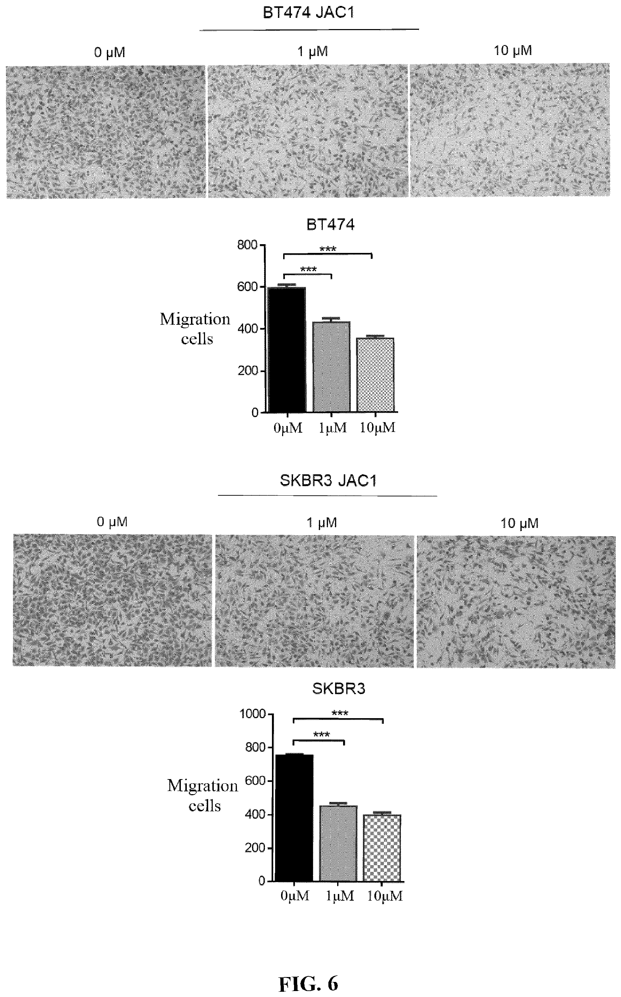

The experimental results are shown in FIG. **6****,** JAC1 can significantly inhibit the migratory ability of cancer breast cells BT474 and SKBR3 and showed to a dose-effect relationship.

It should be noted that: the other compounds prepared in Examples **1-8** were detected with reference to the method of Example **15.** The results were consistent with JAC1 that can significantly inhibit the migratory ability of cancer breast cells BT474 and SKBR3 and showed a dose-effect relationship.

### Example 16

Effect of JAC1 on tumor growth in a model of nude mice subcutaneously transplanted with HER2-positive breast cancer cells.

This example is used to build a model of immunodeficient mice subcutaneously transplanted with human breast cancer cells, and determine the effect of JAC1 on subcutaneous growth of breast cancer cells.

Human breast cancer cells BT474 in the logarithmic growth phase were suspended in ice-cold suspension buffer to a final concentration of 5× 10⁶/100 µL under a sterile condition, followed by subcutaneous transplantation of the suspended cells into BALB/c nude mice. When the tumor grown to 75-125 mm³, the BALB/c nude mice were randomly divided into five groups, including a blank control group (Mock), a solvent control group (Vehicle), a 50 mg/kg JAC1 treatment group, a 100 mg/kg JAC1 treatment group, and a positive control group treated with Pacilitaxel and Trastuzumab (TH). Dose and administration: JAC1 was injected intraperitoneally into mice once daily; Trastuzumab at the dosage of 10 mg/kg was injected intraperitoneally twice a week; Pacilitaxel at the dosage of 20 mg/kg was injected intraperitoneally once a week. From the day of administration, the body weight of mice was weighed every other 2 days, and tumor diameter was observed and measured at the same time to calculate the tumor volume. According to the humane requirements for animals, when the tumor volume of the model mice with tumor reached 2500-3000 mm³, the research based on the mouse models was terminated and the laboratory mice were anesthetized and euthanized for further detection and analysis.

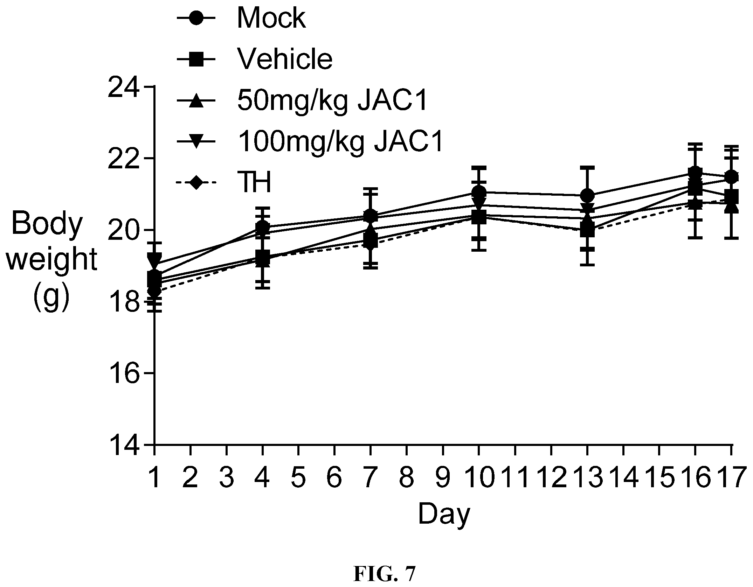

Analysis of the results showed that body weight of each mouse exhibited a slow increasing trend during the experimental phase (FIG. 7).

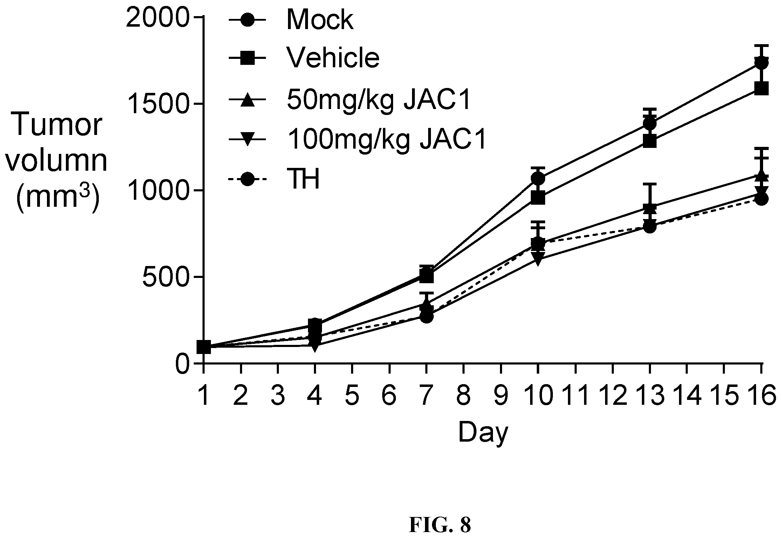

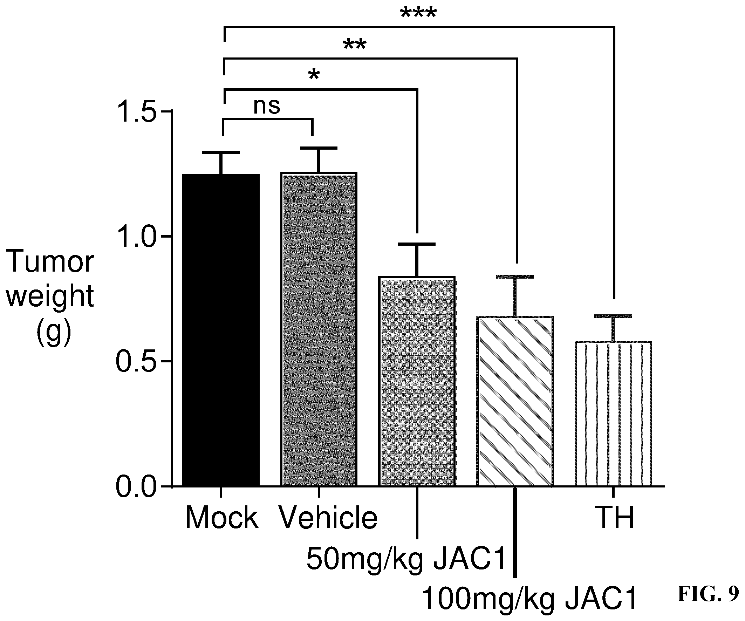

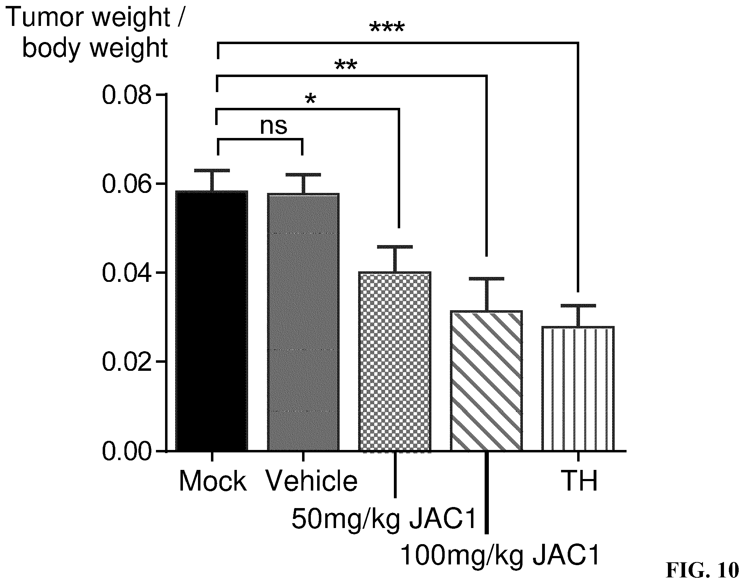

When compared with the blank control group, in the 50 mg/kg JAC1 treatment group, the 100 mg/kg JAC1 treatment group, and the TH group, the growth rate of tumor volume in mice significantly reduced (FIG. **8****),** tumor weight reduced (P <0.05) (the average tumor weight: TH group < 100 mg/kg JAC1 treatment group < 50 mg/kg JAC1 treatment group)(FIG. **9****),** and the tumor weight/ body weight ratio decreased (P<0.05) (the TH group shows the lowest tumor weight/ body weight ratio) (FIG. **10****).**

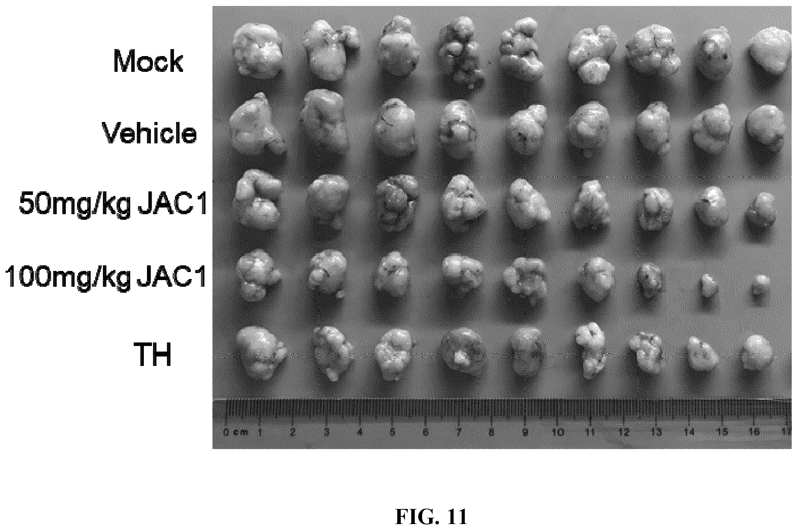

FIG. **11** is a schematic diagram of the tumor tissue isolated from each group of mice.

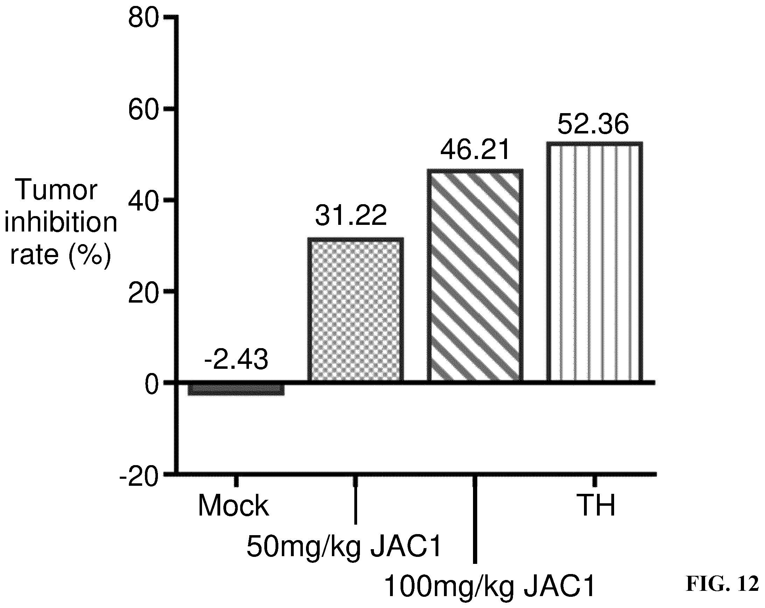

Tumor growth inhibition rate: TH group (52.36%)>100 mg/kg JAC1 group (46.21%) > 50 mg/kg JAC1 group (31.22%) (FIG. **12****);** the results indicated that all of TH, 100 mg/kg JAC1, and 50 mg/kg JAC1 were effective.

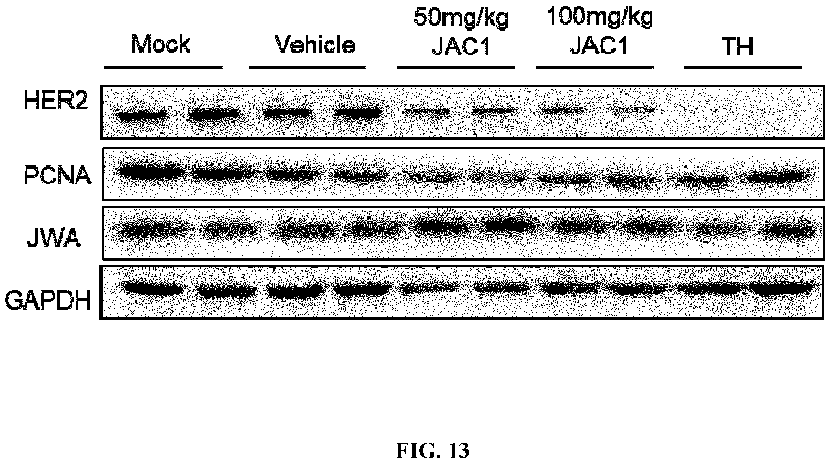

Further, Western blotting was performed to test the expression level of related proteins in tumor tissues of the subcutaneous breast cancer in each treatment group. Referring to FIG. **13****,** in the JAC1 treatment groups, the HER2 expression was decreased significantly, while, conversely, the JWA expression level significantly increased; the HER2 expression of the TH group was not detected in the Western blotting because Trastuzumab was a humanized anti-HER2 antibody that have bound to the epitopes on the HER2 protein so that the primary antibody could not bind to HER2 protein in the Western blotting.

### Example 17

Effect of JAC1 on the major organ structures in mice bearing tumor.

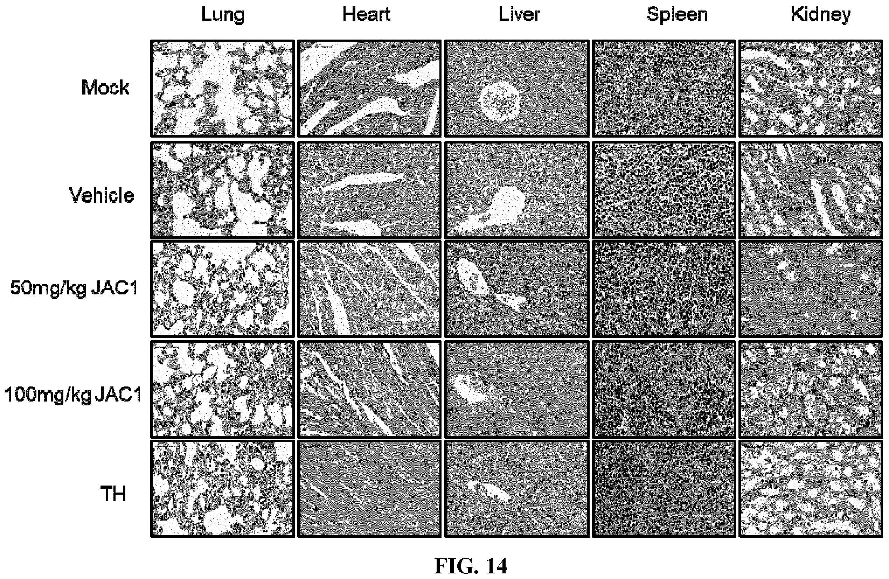

A model of nude mice subcutaneously transplanted with human breast cancer cells, which can be inhibited by JAC1, was built with reference to the method of Example **16.** At the end of the model, in order to observe whether the major organs of laboratory mice were damaged under each treatment condition, the mice were anesthetized and euthanized, and the major organ tissues, such as heart, liver, spleen, lung, kidney, and brain, were fixed in formalin solution using conventional methods. After embedded in paraffin, the organ tissues were sectioned and stained with H&E to observe whether the major organ tissues were normal in morphology. The results indicated that under the experimental condition, no obvious anomalies were observed in the major organs (including heart, liver, spleen, lung, kidney, and brain) of laboratory mice under a microscope (FIG. **14****).**

### Example 18

Effect of JAC1 on the biochemical indexes in blood of mice bearing tumor .

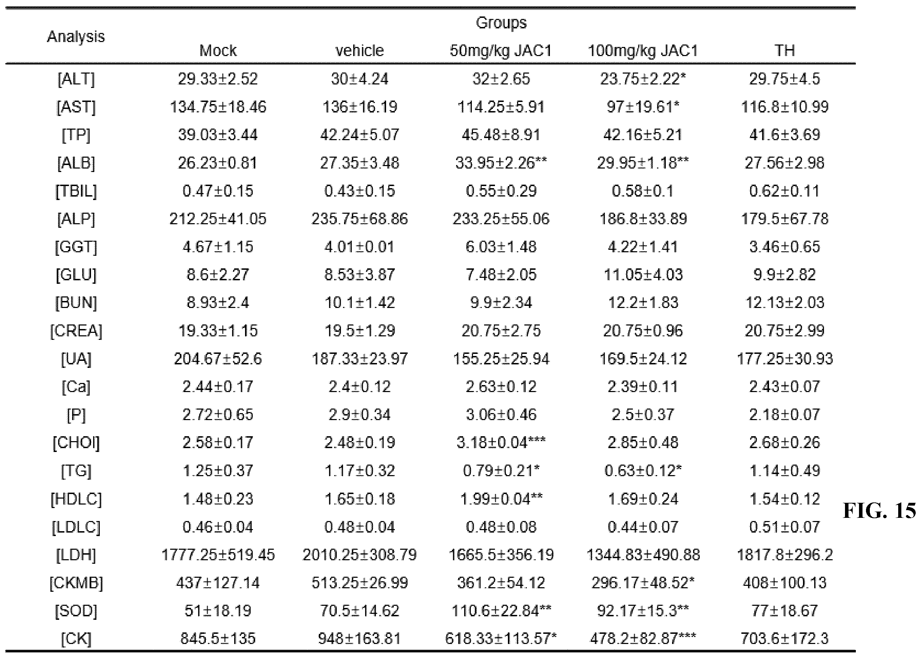

A model of nude mice subcutaneously transplanted with human breast cancer cells, which can be inhibited by JAC1, was built with reference to the method of Example **16.** At the end of the model, in order to observe whether the major organs of laboratory mice were damaged under each treatment condition, the mice were anesthetized and whole blood was collected from the anesthetized mice to separate serum. The change of biochemical indexes in the serum was detected using an automatic biochemistry analyzer. Referring to FIG. **15****,** the results indicated that no obvious morphological anomalies were observed in the structures of the major organs in laboratory mice. However, when compared with the blank control group, in the JAC1 treatment groups, particularly the 100 mg/kg JAC1 treatment group, the liver function aspartate transaminase (AST) and alanine transaminase (ALT), triglyceride (TG), and creatine kinase (CK) and the isoenzyme thereof (CKMB) in the serum of mice significant decreased. Conversely, the level of superoxide dismutase (SOD) as an antioxidant enzyme significantly increased. These indexes were improved in the TH group compared with the blank control group, while showing no statistically significant difference between the two groups.

The results indicated that JAC1 can not only effectively inhibit the growth of tumor in mice subcutaneously transplanted with human breast cancer cells BT474, but also provide a better *in vivo* intervention for the biochemical dysfunction caused by tumors. The positive control group had not significant improving effect on *in vivo* biochemical dysfunction caused by tumors, despite the fact that the positive control group had a slightly higher tumor growth inhibition rate than the JAC1 treatment groups.

### Example 19

Detection of off-target effects occurring when JAC1 activates JWA expression and inhibits HER2 expression.

We further detected the off-target effect of JAC1 on tumor suppression upon observing the specifically inhibitory effect of the small molecule JAC1 on the growth of cancer cells overexpressing HER2. A HER2-positive human gastric cancer cell line BGC823 was established using Crisp/cas9 technology, in which JWA gene was deleted. The human gastric cancer cell line BGC823 (JWA WT) with JWA gene, and the human gastric cancer cell line BGC823 (JWA KO) without JWA gene (both of which had been proved to be HER2-positive), were treated with 10 µM JAC1 for 48 h. After protein extraction, Western blotting technology was used to determine the expression levels of JWA and HER2 in the wild-type cell line and the JWA-knockout cell line of BGC823.

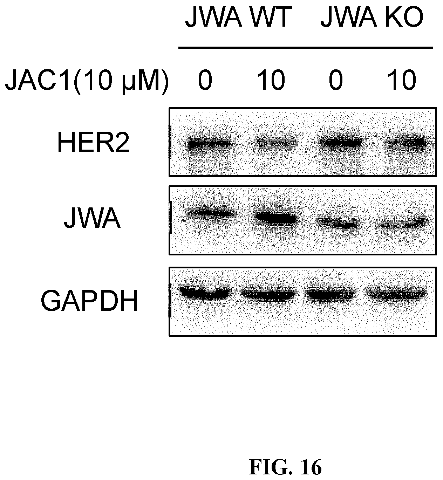

Referring to FIG. **16****,** the results indicated that JAC1 treatment can significantly activate JWA expression and inhibit HER2 expression in the wild-type gastric cancer cell (JWA WT). However, JAC1 treatment had no effect on the expression of JWA and HER2 in the JWA-knockout gastric cancer cell (JWA KO).

The results indicated that the inhibitory effect of JAC1 on HER2 was achieved by activating JWA and JAC1 had no detectable off-target effects.

### Example 20

Many compounds capable of activating the expression of JWA gene in cells were identified by using a high-throughput screening according to the method of Example **11.** In addition to the compounds prepared in Examples **1-8,** other compounds were as follows:
R¹ = OH, R² = H or OH, R³ = F or Cl, R⁴ = H or F, R⁵ = H, CH₃ or CH₂CH₃;
R¹ = CH₃, R² = CH₃ or CH₂CH₃, R³ = I or CF₃, R⁴ = Br or CF₃, R⁵ = H, CH₃ or CH₂CH₃;
R¹ = CH₂CH₃, R² = CH₂CH₂CH₃ or OCH₃, R³ = Br or Cl, R⁴ = Cl or I, R⁵ = H, CH₃ or CH₂CH₃;
R¹ = OCH₃, R² = OCH₂CH₂CH₃ or OH, R³ = F or CF₃, R⁴ = H or CF₃, R⁵ *=* H, CH₃ or CH₂CH₃;
R¹ = OCH₂CH₃; R² = CH₃ or OCH₃, R³ = Cl or I, R⁴ = Br or I, R⁵ = H, CH₃ or CH₂CH₃.

Note: when substituents of the above-mentioned compounds may be present at several substitution positions, or when the above-mentioned compounds are present in the form of several isomers, it means that all possible compounds are included.

The above-mentioned compounds were all capable of activating the expression of JWA gene in cells, and thereby had a prospect of preparing JWA gene activators or antitumor drugs.

In addition, the compounds can also be used in combination with other drugs and other therapeutic means to enhance the efficacy of the treatment for malignant tumors.

## Claims

1. A compound having the structure of Formula I: 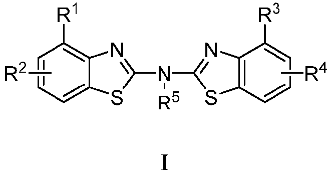 wherein:
R¹ is selected from the group consisting of -OH, -CH₃, -CH₂CH₃, -OCH₃, and - OCH₂CH₃;
R² is selected from the group consisting of -H, -OH, -CH₃, -CH₂CH₃, -CH₂CH₂CH₃, - OCH₃, and - OCH₂CH₂CH₃;
R³ is selected from the group consisting of -Cl, -Br, -I, and -CF3;
R⁴ is selected from the group consisting of -H, -F, -Cl, -Br, -I, and -CF3; and
R⁵ is selected from the group consisting of -H, -CH3, and -CH₂CH₃.

2. A compound, being one of the following compounds:
4,7-difluoro-N-(4-methoxybenzo[d]thiazol-2-yl)benzo[d]thiazol-2-amine;
4-methoxy-N-(4-(trifluoromethyl)benzo[d]thiazol-2-yl)benzo[d]thiazol-2-amine;
N-(4-fluorobenzo[d]thiazol-2-yl)-4,7-dimethoxybenzo[d]thiazol-2-amine;
4,7-dimethoxy-N-(4-(trifluoromethyl)benzo[d]thiazol-2-yl)benzo[d]thiazol-2-amine; and
4,6-dimethoxy-N-(4-(trifluoromethyl)benzo[d]thiazol-2-yl)benzo[d]thiazol-2-amine; the structural formulae thereof being as follows, respectively:

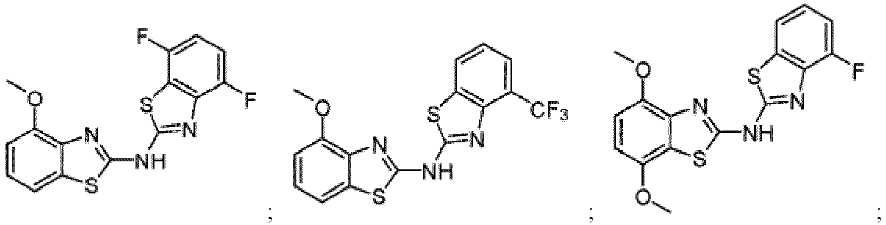

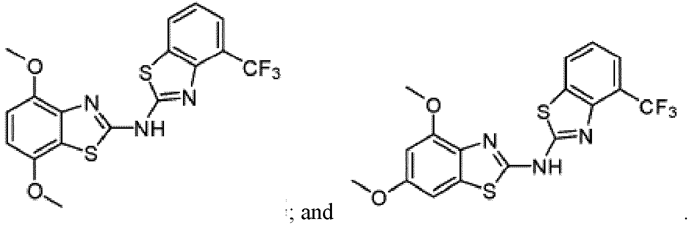

3. A method for preparing the compound of claim 1, comprising the following steps: 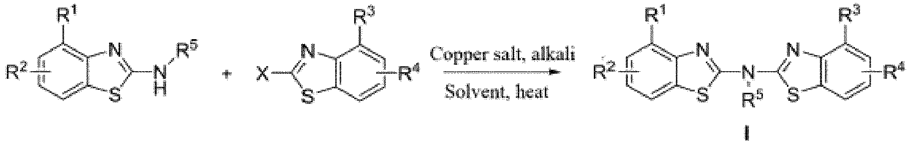 wherein:
X is selected from the group consisting of Cl, Br, and I;
said copper salt is selected from the group consisting of copper oxide, cuprous oxide, copper chloride, cuprous chloride, copper bromide, cuprous bromide and cuprous iodide;
said alkali is selected from the group consisting of sodium hydroxide, potassium hydroxide, potassium carbonate, sodium carbonate, potassium tert-butoxide and sodium hydride;
said solvent is selected from the group consisting of tetrahydrofuran, dimethyl sulfoxide and N,N-dimethylformamide; and
said heating temperature is 80-120°C.

4. The method of claim 3, wherein X is Cl; said copper salt is cuprous iodide; said alkali is potassium carbonate; said solvent is dimethyl sulfoxide; and said heating temperature is 100°C.

5. A pharmaceutical salt of the compound of any one of claims 1 to2.

6. The salt of claim 5, being a salt formed from the compound of formula I with an acid selected from the group consisting of hydrochloric acid, sulfuric acid, benzenesulfonic acid, p-toluenesulfonic acid, phosphoric acid, hydrobromic acid, maleic acid, fumaric acid and malic acid.

7. A pharmaceutical composition comprising a compound having the structure of Formula I: 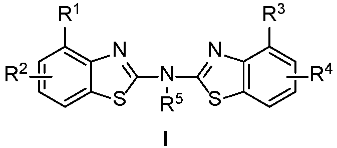 wherein:
R¹ is selected from the group consisting of -OH, -CH3, -CH2CH3, -OCH3, and -OCH2CH3;
R2 is selected from the group consisting of -H, -OH, -CH3, -CH2CH3, -CH2CH2CH3, -OCH3, and -OCH2CH2CH3;
R3 is selected from the group consisting of -F, -Cl, -Br, -I, and -CF3;
R4 is selected from the group consisting of -H, -F, -Cl, -Br, -I, and -CF3; and
R5 is selected from the group consisting of -H, -CH3, and -CH2CH3;
or a pharmaceutical salt of the compound having the structure of Formula I.

8. The pharmaceutical composition of claim 7, wherein R¹ is -OCH₃; R² is -H or -OCH₃; R³ is -F, or -CF₃; R⁴ is -H or -F; and R⁵ is -H.

9. The pharmaceutical composition of claim 7,
wherein the compound is selected from the group consisting of: 4-fluoro-N-(4-methoxybenzo[d]thiazol-2-yl)benzo[d]thiazol-2-amine; 4,7-difluoro-N-(4-methoxybenzo[d]thiazol-2-yl)benzo[d]thiazol-2-amine; 4,6-difluoro-N-(4-methoxylbenzo[d]thiazol-2-yl)benzo[d]thiazol-2-amine; 4-methoxy-N-(4-(trifluoromethyl)benzo[d]thiazol-2-yl)benzo[d]thiazol-2-amine; N-(4-fluorobenzo[d]thiazol-2-yl)-4,7-dimethoxybenzo[d]thiazol-2-amine; N-(4-fluorobenzo[d]thiazol-2-yl)-4,6-dimethoxybenzo[d]thiazol-2-amine; 4,7-dimethoxy-N-(4-(trifluoromethyl)benzo[d]thiazol-2-yl)benzo[d]thiazol-2-amine; and 4,6-dimethoxy-N-(4-(trifluoromethyl)benzo[d]thiazol-2-yl)benzo[d]thiazol-2-amine; the structural formulae thereof being as follows, respectively:

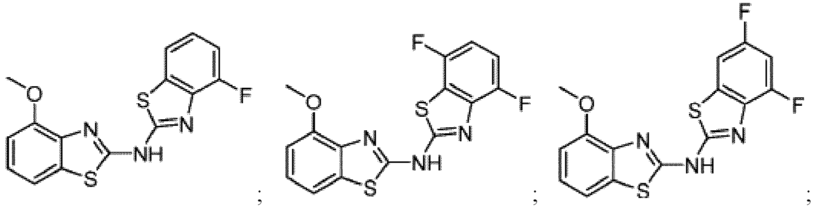

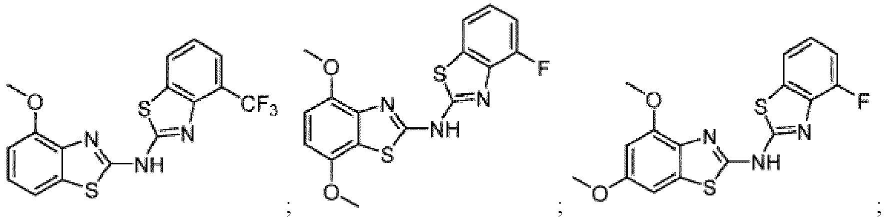

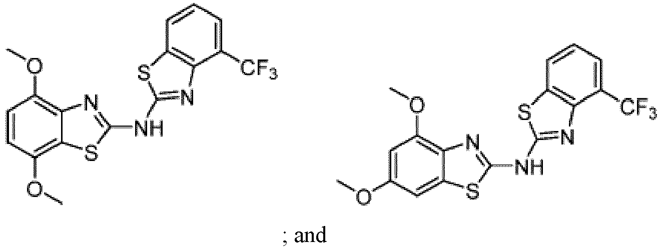

10. A compound of claim 1 or 2, or a pharmaceutical salt of claim 5 or 6, or a pharmaceutical composition of any one of claims 7 to 9, for use in the treatment of tumors.

11. The compound for use or the pharmaceutical salt for use or the pharmaceutical composition for use of claim 10, wherein the tumors are those associated with low expression of JWA gene and overexpression of HER2, and the tumors include breast cancer, gastric cancer, lung cancer, and glioma; and the compound, the pharmaceutical salt or the pharmaceutical composition inhibits the tumors by activating the expression of the JWA protein and then degrading the HER2 protein.

12. A compound of claim 1 or 2, or a pharmaceutical salt of claim 5 or 6, or a pharmaceutical composition of any one of claims 7 to 9, for use as a JWA gene activator medicament.

13. A compound having the structure of Formula I for use as an antitumor medicament, 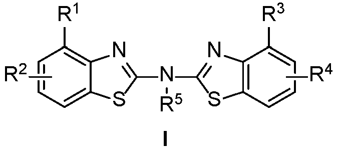 wherein:
R¹ is selected from the group consisting of -OH, -CH₃, -CH₂CH₃, -OCH₃, and - OCH₂CH₃;
R² is selected from the group consisting of -H, -OH, -CH₃, -CH₂CH₃, -CH₂CH₂CH₃, - OCH₃, and -OCH₂CH₂CH₃;
R³ is selected from the group consisting of -F, -Cl, -Br, -I, and -CF₃;
R⁴ is selected from the group consisting of -H, -F, -Cl, -Br, -I, and -CF₃; and
R⁵ is selected from the group consisting of -H, -CH₃, and -CH₂CH₃;
or a pharmaceutical salt of the compound having the structure of Formula I.

14. The compound for use of claim 13, wherein R¹ is -OCH₃; R² is -H or -OCH₃; R³ is -F, or -CF₃; R⁴ is -H or -F; and R⁵ is -H.

15. The compound for use of claim 13, wherein the compound is selected from the group consisting of:
4-fluoro-N-(4-methoxybenzo[d]thiazol-2-yl)benzo[d]thiazol-2-amine;
4,7-difluoro-N-(4-methoxybenzo[d]thiazol-2-yl)benzo[d]thiazol-2-amine;
4,6-difluoro-N-(4-methoxylbenzo[d]thiazol-2-yl)benzo[d]thiazol-2-amine;
4-methoxy-N-(4-(trifluoromethyl)benzo[d]thiazol-2-yl)benzo[d]thiazol-2-amine;
N-(4-fluorobenzo[d]thiazol-2-yl)-4,7-dimethoxybenzo[d]thiazol-2-amine;
N-(4-fluorobenzo[d]thiazol-2-yl)-4,6-dimethoxybenzo[d]thiazol-2-amine;
4,7-dimethoxy-N-(4-(trifluoromethyl)benzo[d]thiazol-2-yl)benzo[d]thiazol-2-amine; and
4,6-dimethoxy-N-(4-(trifluoromethyl)benzo[d]thiazol-2-yl)benzo[d]thiazol-2-amine;
the structural formulae thereof being as follows, respectively:

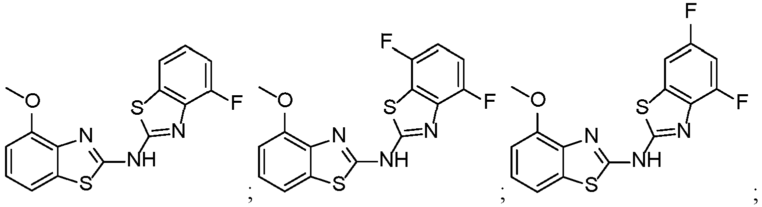

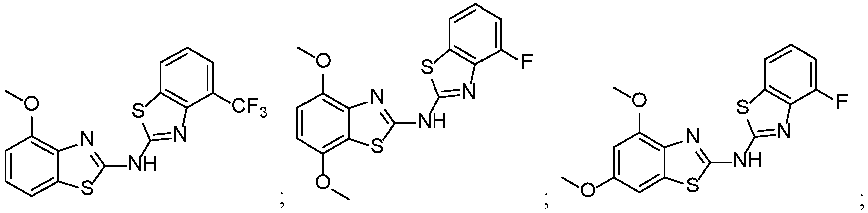

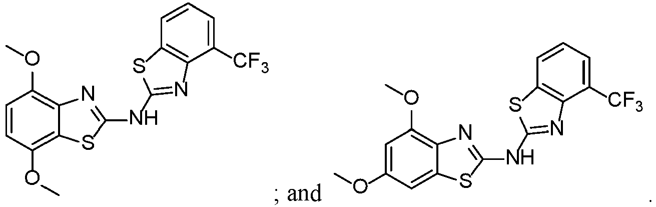

## Patentansprüche

1. Verbindung mit der Struktur der Formel I: 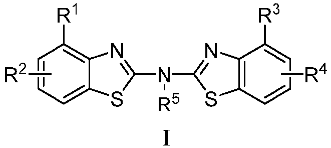 wobei:
R¹ ausgewählt ist aus der Gruppe bestehend aus -OH, -CH₃, -CH₂CH₃, -OCH₃, und -OCH₂CH₃;
R² ausgewählt ist aus der Gruppe bestehend aus -H, -OH, -CH₃, -CH₂CH₃, -CH₂CH₂CH₃, -OCH₃ und - OCH₂CH₂CH₃,
R³ ausgewählt ist aus der Gruppe bestehend aus -Cl, -Br, -I und -CF₃;
R⁴ ausgewählt ist aus der Gruppe bestehend aus -H, -F, -Cl, -Br, -I und -CF₃; und
R⁵ ausgewählt ist aus der Gruppe bestehend aus -H, -CH₃ und -CH₂CH₃.

2. Verbindung, die eine der folgenden Verbindungen ist:
4,7-Difluor-N-(4-methoxybenzo[d]thiazol-2-yl)benzo[d]thiazol-2-amin;
4-Methoxy-N-(4-(trifluormethyl)benzo[d]thiazol-2-yl)benzo[d]thiazol-2-amin;
N-(4-Fluorbenzo[d]thiazol-2-yl)-4,7-dimethoxybenzo[d]thiazol-2-amin;
4,7-Dimethoxy-N-(4-(trifluormethyl)benzo[d]thiazol-2-yl)benzo[d]thiazol-2-amin; und
4,6-Dimethoxy-N-(4-(trifluormethyl)benzo[d]thiazol-2-yl)benzo[d]thiazol-2-amin; die Strukturformeln davon jeweils wie folgt sind: 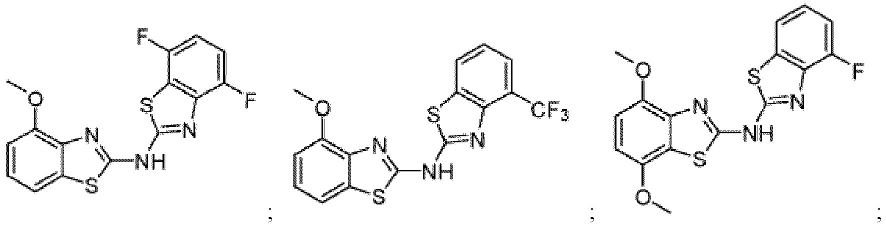 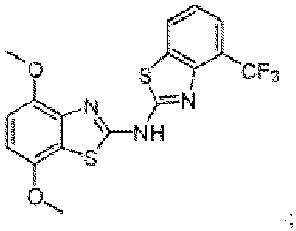 und

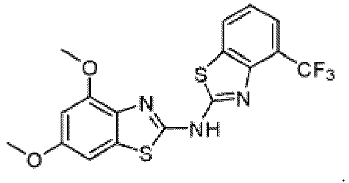

3. Verfahren zur Herstellung der Verbindung nach Anspruch 1, umfassend die folgenden Schritte: 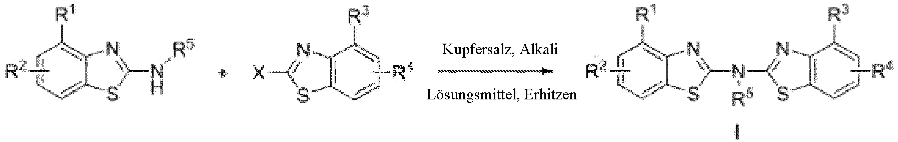 wobei:
X ausgewählt ist aus der Gruppe bestehend aus Cl, Br und I;
das Kupfersalz ausgewählt ist aus der Gruppe bestehend aus Kupferoxid, Kupfer(I)-oxid, Kupferchlorid, Kupfer(I)-chlorid, Kupferbromid, Kupfer(I)-bromid und Kupfer(I)-iodid;
das Alkali ausgewählt ist aus der Gruppe bestehend aus Natriumhydroxid, Kaliumhydroxid, Kaliumcarbonat, Natriumcarbonat, Kalium-tert-butoxid und Natriumhydrid;
das Lösungsmittel ausgewählt ist aus der Gruppe bestehend aus Tetrahydrofuran, Dimethylsulfoxid und N,N-Dimethylformamid; und
die Heiztemperatur 80-120 °C beträgt.

4. Verfahren nach Anspruch 3, wobei X Cl ist; das Kupfersalz Kupfer(I)-iodid ist; das Alkali Kaliumcarbonat ist; das Lösungsmittel Dimethylsulfoxid ist; und die Heiztemperatur 100 °C beträgt.

5. Pharmazeutisches Salz der Verbindung nach einem der Ansprüche 1 bis 2.

6. Salz nach Anspruch 5, das ein Salz ist, das aus der Verbindung der Formel I mit einer Säure gebildet wird, die ausgewählt ist aus der Gruppe bestehend aus Salzsäure, Schwefelsäure, Benzolsulfonsäure, p-Toluolsulfonsäure, Phosphorsäure, Bromwasserstoffsäure, Maleinsäure, Fumarsäure und Äpfelsäure.

7. Pharmazeutische Zusammensetzung, umfassend eine Verbindung mit der Struktur der Formel I: 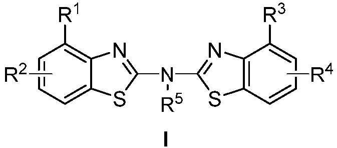 wobei:
R¹ ausgewählt ist aus der Gruppe bestehend aus -OH, -CH₃, -CH₂CH₃, -OCH₃ und -OCH₂CH₃;
R² ausgewählt ist aus der Gruppe bestehend aus -H, -OH, -CH₃, -CH₂CH₃, -CH₂CH₂CH₃, -OCH₃ und -OCH₂CH₂CH₃;
R³ ausgewählt ist aus der Gruppe bestehend aus -F, -Cl, -Br, -I und -CF₃;
R⁴ ausgewählt ist aus der Gruppe bestehend aus -H, -F, -Cl, -Br, -I und -CF₃; und
R⁵ ausgewählt ist aus der Gruppe bestehend aus -H, -CH₃ und -CH₂CH₃,
oder ein pharmazeutisches Salz der Verbindung mit der Struktur der Formel I.

8. Pharmazeutische Zusammensetzung nach Anspruch 7, wobei R¹ -OCH₃ ist; R² -H oder -OCH₃ ist; R³ -F oder -CF₃ ist; R⁴ -H oder -F ist; und R⁵ -H ist.

9. Pharmazeutische Zusammensetzung nach Anspruch 7,
wobei die Verbindung ausgewählt ist aus der Gruppe bestehend aus: 4-Fluor-N-(4-methoxybenzo[d]thiazol-2-yl)benzo[d]thiazol-2-amin; 4,7-Difluor-N-(4-methoxybenzo[d]thiazol-2-yl)benzo[d]thiazol-2-amin; 4,6-Difluor-N-(4-methoxybenzo[d]thiazol-2-yl)benzo[d]thiazol-2-amin; 4-Methoxy-N-(4-(trifluormethyl)benzo[d]thiazol-2-yl)benzo[d]thiazol-2-amin; N-(4-Fluorbenzo[d]thiazol-2-yl)-4,7-dimethoxybenzo[d]thiazol-2-amin; N-(4-Fluorbenzo[d]thiazol-2-yl)-4,6-dimethoxybenzo[d]thiazol-2-amin; 4,7-Dimethoxy-N-(4-(trifluormethyl)benzo[d]thiazol-2-yl)benzo[d]thiazol-2-amin; und 4,6-Dimethoxy-N-(4-(trifluormethyl)benzo[d]thiazol-2-yl)benzo[d]thiazol-2-amin; die Strukturformeln davon jeweils wie folgt sind:

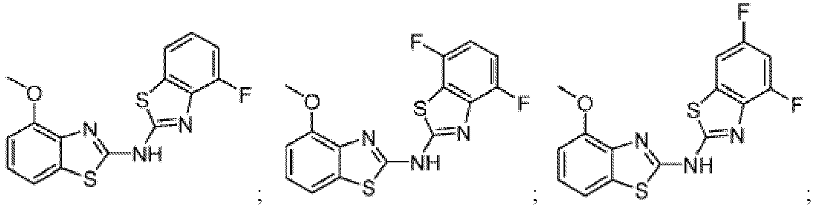

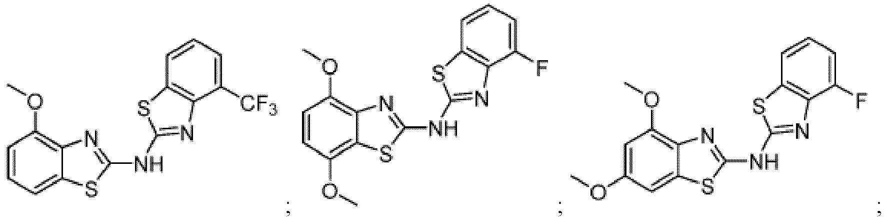

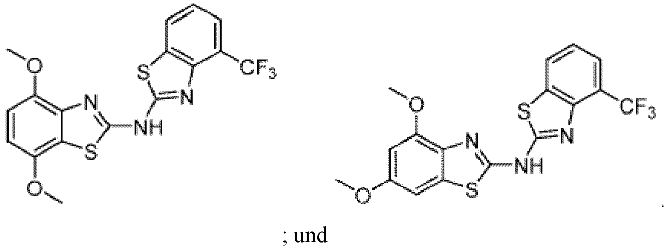

10. Verbindung nach Anspruch 1 oder 2 oder pharmazeutisches Salz nach Anspruch 5 oder 6 oder pharmazeutische Zusammensetzung nach einer der Ansprüche 7 bis 9, zur Verwendung bei der Behandlung von Tumoren.

11. Verbindung zur Verwendung oder pharmazeutisches Salz zur Verwendung oder pharmazeutische Zusammensetzung zur Verwendung nach Anspruch 10, wobei es sich bei den Tumoren um solche handelt, die mit einer geringen Expression des JWA-Gens und einer Überexpression von HER2 assoziiert sind, und die Tumoren Brustkrebs, Magenkrebs, Lungenkrebs und Gliome einschließen; und die Verbindung, das pharmazeutische Salz oder die pharmazeutische Zusammensetzung die Tumoren hemmt, indem sie die Expression des JWA-Proteins aktiviert und dann das HER2-Protein abbaut.

12. Verbindung nach Anspruch 1 oder 2 oder pharmazeutisches Salz nach Anspruch 5 oder 6 oder pharmazeutische Zusammensetzung nach einem der Ansprüche 7 bis 9 zur Verwendung als JWA-Genaktivatormedikament.

13. Verbindung mit der Struktur der Formel I zur Verwendung als Antitumormedikament, 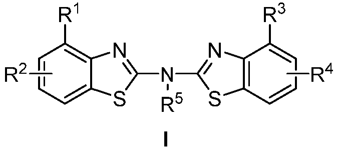 wobei:
R¹ ausgewählt ist aus der Gruppe bestehend aus -OH, -CH₃, -CH₂CH₃, -OCH₃, und - OCH₂CH₃;
R² ausgewählt ist aus der Gruppe bestehend aus -H, -OH, -CH₃, -CH₂CH₃, -CH₂CH₂CH₃, - OCH₃und -OCH₂CH₂CH₃;
R³ ausgewählt ist aus der Gruppe bestehend aus -F, -Cl, -Br, -I und -CF₃;
R⁴ ausgewählt ist aus der Gruppe bestehend aus -H, -F, -Cl, -Br, -I und -CF₃; und
R⁵ ausgewählt ist aus der Gruppe bestehend aus -H, -CH₃ und -CH₂CH₃,
oder pharmazeutisches Salz der Verbindung mit der Struktur der Formel I.

14. Verbindung zur Verwendung nach Anspruch 13, wobei R¹ -OCH₃ ist; R² -H oder -OCH₃ ist; R³ -F oder -CF₃ ist; R⁴ -H oder -F ist; und R⁵ -H ist.

15. Verbindung zur Verwendung nach Anspruch 13, wobei die Verbindung ausgewählt ist aus der Gruppe bestehend aus:
4-Fluor-N-(4-methoxybenzo[d]thiazol-2-yl)benzo[d]thiazol-2-amin;
4,7-Difluor-N-(4-methoxybenzo[d]thiazol-2-yl)benzo[d]thiazol-2-amin;
4,6-Difluor-N-(4-methoxybenzo[d]thiazol-2-yl)benzo[d]thiazol-2-amin;
4-Methoxy-N-(4-(trifluormethyl)benzo[d]thiazol-2-yl)benzo[d]thiazol-2-amin;
N-(4-Fluorbenzo[d]thiazol-2-yl)-4,7-dimethoxybenzo[d]thiazol-2-amin;
N-(4-Fluorbenzo[d]thiazol-2-yl)-4,6-dimethoxybenzo[d]thiazol-2-amin;
4,7-Dimethoxy-N-(4-(trifluormethyl)benzo[d]thiazol-2-yl)benzo[d]thiazol-2-amin; und
4,6-Dimethoxy-N-(4-(trifluormethyl)benzo[d]thiazol-2-yl)benzo[d]thiazol-2-amin;
die Strukturformeln davon jeweils wie folgt sind:

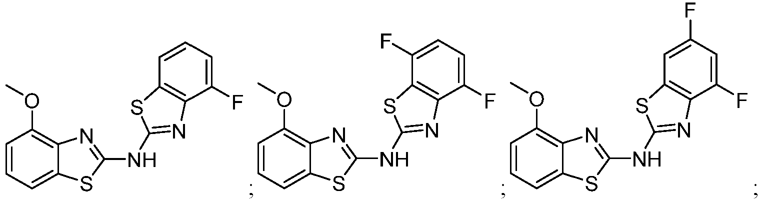

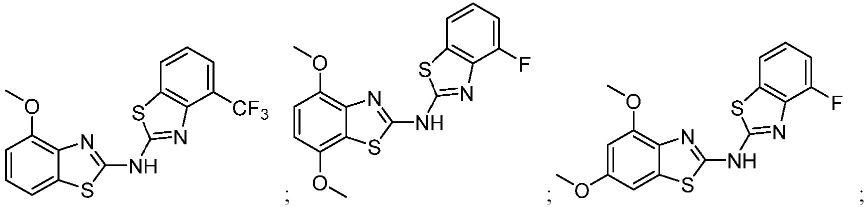

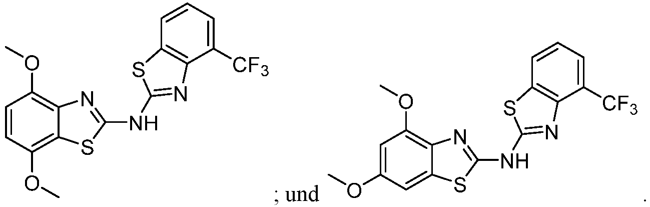

## Revendications

1. Composé ayant la structure de formule I : 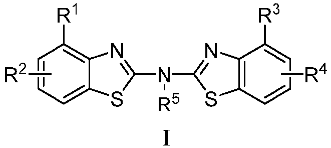 dans lequel :
R¹ est choisi dans le groupe constitué de -OH, -CH₃, -CH₂CH₃, -OCH₃, et - OCH₂CH₃ ;
R² est choisi dans le groupe constitué de -H, -OH, -CH₃, -CH₂CH₃, -CH₂CH₂CH₃, - OCH₃, et - OCH₂CH₂CH₃ ;
R³ est choisi dans le groupe constitué de -Cl, -Br, -I et -CF₃ ;
R⁴ est choisi dans le groupe constitué de -H, -F, -Cl, -Br, -I et -CF₃ ; et
R⁵ est choisi dans le groupe constitué de -H, -CH₃ et -CH₂CH₃.

2. Composé, qui est l'un des composés suivants :
4,7-difluoro-N-(4-méthoxybenzo[d]thiazol-2-yl)benzo[d]thiazol-2-amine ;
4-méthoxy-N-(4-(trifluorométhyl)benzo[d]thiazol-2-yl)benzo[d]thiazol-2-amine ;
N-(4-fluorobenzo[d]thiazol-2-yl)-4,7-diméthoxybenzo[d]thiazol-2-amine ;
4,7-diméthoxy-N-(4-(trifluorométhyl)benzo[d]thiazol-2-yl)benzo[d]thiazol-2-amine ; et
4,6-diméthoxy-N-(4-(trifluorométhyl)benzo[d]thiazol-2-yl)benzo[d]thiazol-2-amine ; les formules développées de ceux-ci étant respectivement les suivantes :

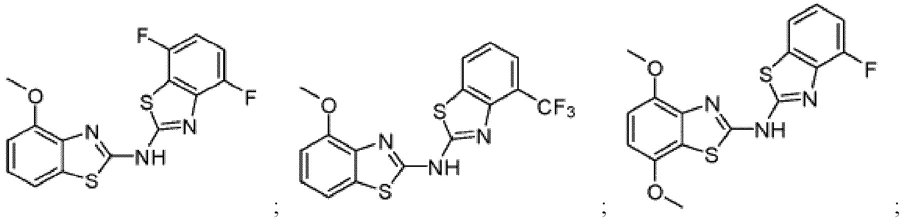

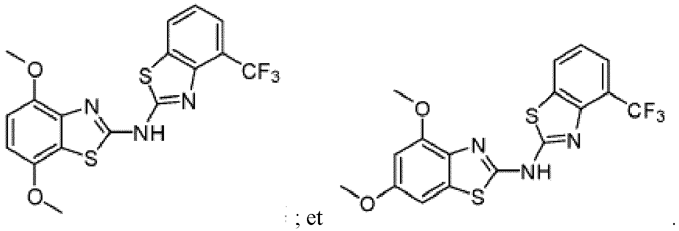

3. Procédé de préparation du composé selon la revendication 1, comprenant les étapes suivantes : 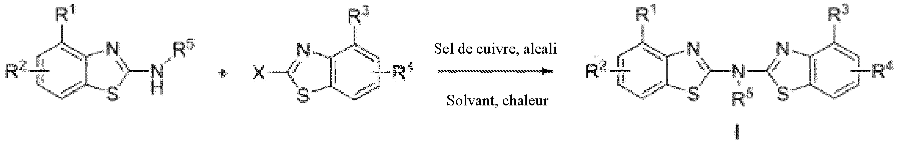 dans lequel :
X est choisi dans le groupe constitué de Cl, Br et I ;
ledit sel de cuivre est choisi dans le groupe constitué de l'oxyde de cuivre, l'oxyde cuivreux, le chlorure de cuivre, le chlorure cuivreux, le bromure de cuivre, le bromure cuivreux et l'iodure cuivreux ;
ledit alcali est choisi dans le groupe constitué de l'hydroxyde de sodium, l'hydroxyde de potassium, le carbonate de potassium, le carbonate de sodium, le tert-butylate de potassium et l'hydrure de sodium ;
ledit solvant est choisi dans le groupe constitué du tétrahydrofurane, du diméthylsulfoxyde et du N,N-diméthylformamide ; et
ladite température de chauffage est de 80 à 120 °C.

4. Procédé selon la revendication 3, dans lequel X est Cl ; ledit sel de cuivre est l'iodure cuivreux ; ledit alcali est le carbonate de potassium ; ledit solvant est le diméthylsulfoxyde ; et ladite température de chauffage est de 100 °C.

5. Sel pharmaceutique du composé selon l'une quelconque des revendications 1 à 2.

6. Sel selon la revendication 5, qui est un sel formé à partir du composé de formule I avec un acide choisi dans le groupe constitué de l'acide chlorhydrique, acide sulfurique, acide benzènesulfonique, acide p-toluènesulfonique, acide phosphorique, acide bromhydrique, acide maléique, acide fumarique et acide malique.

7. Composition pharmaceutique comprenant un composé ayant la structure de formule I : 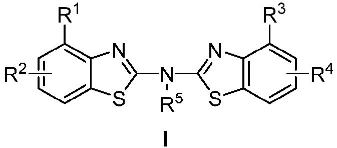 dans laquelle :
R¹ est choisi dans le groupe constitué de -OH, -CH₃, -CH₂CH₃, -OCH₃ et -OCH₂CH₃ ;
R² est choisi dans le groupe constitué de -H, -OH, -CH₃, -CH₂CH₃, -CH₂CH₂CH₃, -OCH₃ et - OCH₂CH₂CH₃ ;
R³ est choisi dans le groupe constitué de -F, -Cl, -Br, -I et -CF₃ ;
R⁴ est choisi dans le groupe constitué de -H, -F, -Cl, -Br, -I et -CF₃ ; et
R⁵ est choisi dans le groupe constitué de -H, -CH₃ et -CH₂CH₃ ;
ou un sel pharmaceutique du composé ayant la structure de formule I.

8. Composition pharmaceutique selon la revendication 7, dans laquelle R¹ est -OCH₃ ; R² est - H ou -OCH₃ ; R³ est -F ou -CF₃ ; R⁴ est -H ou -F ; et R⁵ est -H.

9. Composition pharmaceutique selon la revendication 7,
dans laquelle le composé est choisi dans le groupe constitué de : 4-fluoro-N-(4-méthoxybenzo[d]thiazol-2-yl)benzo[d]thiazol-2-amine ; 4,7-difluoro-N-(4-méthoxybenzo[d]thiazol-2-yl)benzo[d]thiazol-2-amine ; 4,6-difluoro-N-(4-méthoxybenzo[d]thiazol-2-yl)benzo[d]thiazol-2-amine ; 4-méthoxy-N-(4-(trifluorométhyl)benzo[d]thiazol-2-yl)benzo[d]thiazol-2-amine ; N-(4-fluorobenzo[d]thiazol-2-yl)-4,7-diméthoxybenzo[d]thiazol-2-amine ; N-(4-fluorobenzo[d]thiazol-2-yl)-4,6-diméthoxybenzo[d]thiazol-2-amine ; 4,7-diméthoxy-N-(4-(trifluorométhyl)benzo[d]thiazol-2-yl)benzo[d]thiazol-2-amine ; et 4,6-diméthoxy-N-(4-(trifluorométhyl)benzo[d]thiazol-2-yl)benzo[d]thiazol-2-amine ; les formules développées de ceux-ci étant respectivement les suivantes :

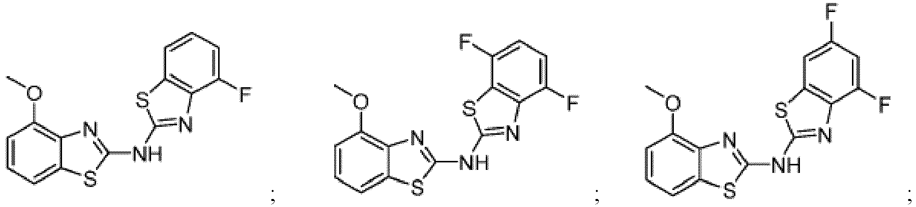

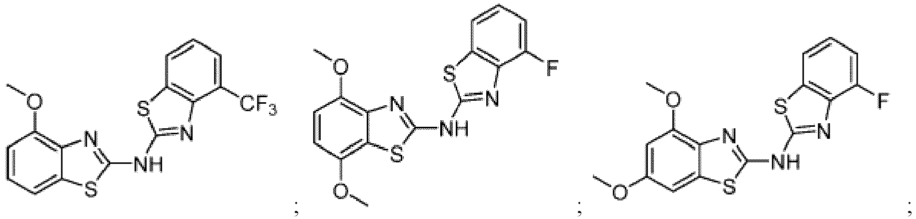

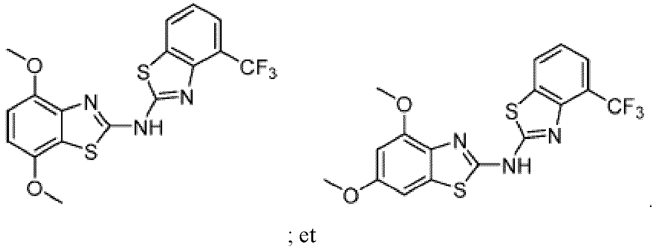

10. Composé selon la revendication 1 ou 2, ou sel pharmaceutique selon la revendication 5 ou 6, ou composition pharmaceutique selon l'une quelconque des revendications 7 à 9, pour une utilisation dans le traitement des tumeurs.

11. Composé pour une utilisation ou sel pharmaceutique pour une utilisation ou composition pharmaceutique pour une utilisation selon la revendication 10, dans lesquels les tumeurs sont celles associées à une faible expression du gène JWA et à une surexpression de HER2, et les tumeurs incluent le cancer du sein, le cancer gastrique, le cancer du poumon et le gliome ; et le composé, le sel pharmaceutique ou la composition pharmaceutique inhibe les tumeurs en activant l'expression de la protéine JWA puis en dégradant la protéine HER2.

12. Composé selon la revendication 1 ou 2, ou sel pharmaceutique selon la revendication 5 ou 6, ou composition pharmaceutique selon l'une quelconque des revendications 7 à 9, pour une utilisation en tant que médicament activateur du gène JWA.

13. Composé ayant la structure de formule I pour une utilisation en tant que médicament antitumoral, 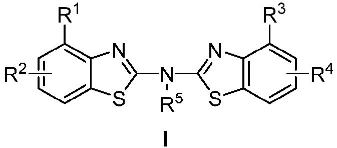 dans lequel :
R¹ est choisi dans le groupe constitué de -OH, -CH₃, -CH₂CH₃, -OCH₃, et -OCH₂CH₃ ;
R² est choisi dans le groupe constitué de -H, -OH, -CH₃, -CH₂CH₃, -CH₂CH₂CH₃, - OCH₃, et -OCH₂CH₂CH₃ ;
R³ est choisi dans le groupe constitué de -F, -Cl, -Br, -I et -CF₃ ;
R⁴ est choisi dans le groupe constitué de -H, -F, -Cl, -Br, -I et -CF₃ ; et
R⁵ est choisi dans le groupe constitué de -H, -CH₃, et -CH₂CH₃ ;
ou un sel pharmaceutique du composé ayant la structure de formule I.

14. Composé pour une utilisation selon la revendication 13, dans lequel R¹ est -OCH₃ ; R² est -H ou -OCH₃ ; R³ est -F ou -CF₃ ; R⁴ est -H ou -F ; et R⁵ est -H.

15. Composé pour une utilisation selon la revendication 13, dans lequel le composé est choisi dans le groupe constitué de :
4-fluoro-N-(4-méthoxybenzo[d]thiazol-2-yl)benzo[d]thiazol-2-amine ;
4,7-difluoro-N-(4-méthoxybenzo[d]thiazol-2-yl)benzo[d]thiazol-2-amine ;
4,6-difluoro-N-(4-méthoxybenzo[d]thiazol-2-yl)benzo[d]thiazol-2-amine ;
4-méthoxy-N-(4-(trifluorométhyl)benzo[d]thiazol-2-yl)benzo[d]thiazol-2-amine ;
N-(4-fluorobenzo[d]thiazol-2-yl)-4,7-diméthoxybenzo[d]thiazol-2-amine ;
N-(4-fluorobenzo[d]thiazol-2-yl)-4,6-diméthoxybenzo[d]thiazol-2-amine ;
4,7-diméthoxy-N-(4-(trifluorométhyl)benzo[d]thiazol-2-yl)benzo[d]thiazol-2-amine ; et
4,6-diméthoxy-N-(4-(trifluorométhyl)benzo[d]thiazol-2-yl)benzo[d]thiazol-2-amine ;
les formules développées de ceux-ci étant respectivement les suivantes :
